(19) Europäisches Patentamt
European Patent Office
Office européen des brevets

(11) EP 1 589 118 A2

(12) EUROPEAN PATENT APPLICATION

(43) Date of publication:
26.10.2005 Bulletin 2005/43

(51) Int Cl.[7]: C12Q 1/68

(21) Application number: 05252153.1

(22) Date of filing: 06.04.2005

(84) Designated Contracting States:
AT BE BG CH CY CZ DE DK EE ES FI FR GB GR
HU IE IS IT LI LT LU MC NL PL PT RO SE SI SK TR
Designated Extension States:
AL BA HR LV MK YU

(30) Priority: 20.04.2004 US 828986

(71) Applicant: Agilent Technologies, Inc.
Palo Alto, CA 94306 (US)

(72) Inventors:
- Barrett, Michael T.
  Mountain View, CA 94040 (US)
- Scheffer, Alicia F.
  Redwood City, CA 94061 (US)

(74) Representative: Tollett, Ian et al
Williams Powell
Morley House
26-30 Holborn Viaduct
London EC1A 2BP (GB)

# (54) Methods and compositions for assessing CpG methylation

(57) Methods and compositions for assessing CpG island methylation are provided. Specifically, the invention provides an unstructured nucleic acid (UNA) oligonucleotide that base pairs with, i.e., hybridizes to, CpG islands. The subject oligonucleotide may be present in an array, and find use in methods for evaluating methylation of CpG islands in cells. Kits and computer programming for use in practicing the subject methods are also provided.

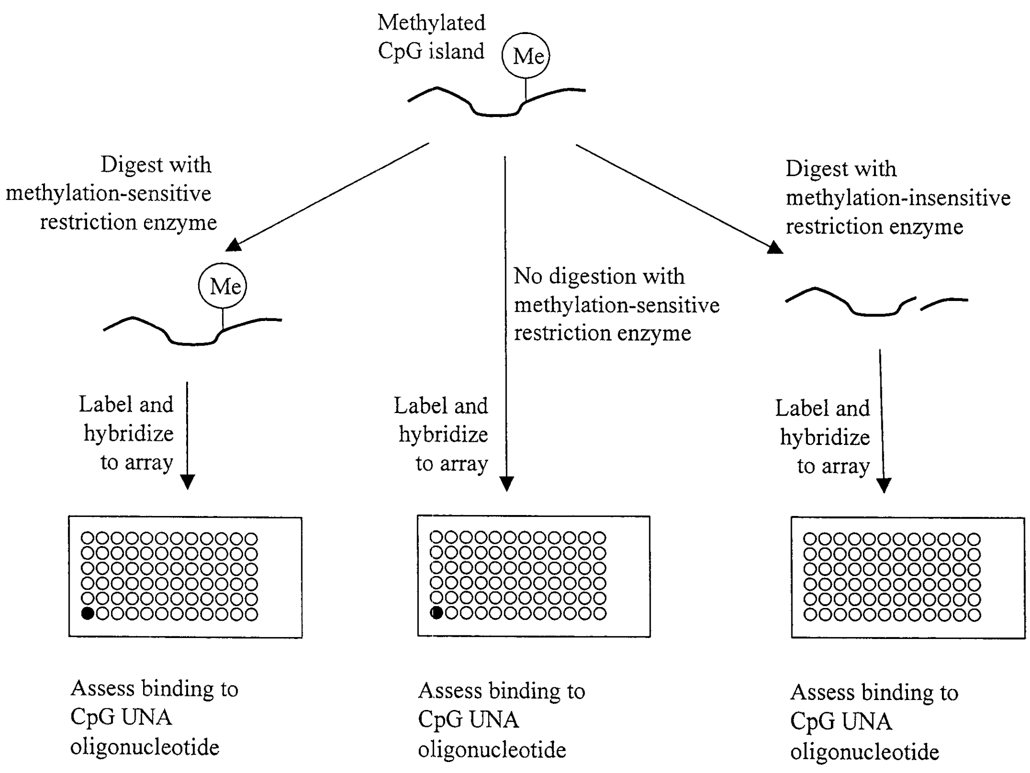


FIG. 2

EP 1 589 118 A2

SEQUENCE LISTING

<110> Agilent Technologies, Inc.

<120> Methods and compositions for assessing CpG methylation

<130> IT/KE/N16773

<150> US 10/828,986
<151> 2004-04-20

<160> 16

<170> FastSEQ for Windows Version 4.0

<210> 1
<211> 139
<212> DNA
<213> Homo sapiens

<400> 1
aaaaagtaca catataacat ttacaggggc tcaaactgcc gctgcctcct ctccggagcg 60
gtccggcctg ccggccgcgg ttccctctcg atgcttcagg gaaccaggac agaaaggtcc 120
ttccgctagc tgtcaggtg 139

<210> 2
<211> 122
<212> DNA
<213> Homo sapiens

<400> 2
acacatataa catttacagg ggctcaaact gccgctgcct cctctccgga gcggtccggc 60
ctgccggccg cggttccctc tcgatgcttc agggaaccag gacagaaagg tccttccgct 120
ag 122

<210> 3
<211> 122
<212> DNA
<213> Homo sapiens

<400> 3
acacatataa catttacagg ggctcaaact gccgctgcct cctctccgga gcggtccggc 60
ctgccggccg cggttccctc tcgatgcttc agggaaccag gacagaaagg tccttccgct 120
ag 122

<210> 4
<211> 99
<212> DNA
<213> Homo sapiens

<400> 4
gggctcaaac tgccgctgcc tcctctccgg agcggtccgg cctgccggcc gcggttccct 60
ctcgatgctt cagggaacca ggacagaaag gtccttccg 99

<210> 5
<211> 122
<212> DNA
<213> Homo sapiens

<400> 5
acacatataa catttacagg ggctcaaact gccgctgcct cctctccgga gcggtccggc 60
ctgccggccg cggttccctc tcgatgcttc agggaaccag gacagaaagg tccttccgct 120

ag 122

<210> 6
<211> 139
<212> DNA
<213> Homo sapiens

<400> 6
aaaaagtaca catataacat ttacaggggc tcaaactgcc gctgcctcct ctccggagcg 60
gtccggcctg ccggccgcgg ttccctctcg atgcttcagg gaaccaggac agaaaggtcc 120
ttccgctagc tgtcaggtg 139

<210> 7
<211> 47
<212> DNA
<213> Homo sapiens

<400> 7
acacatataa catttacagg ggctcaaact gccgctgcct cctctcc 47

<210> 8
<211> 10
<212> DNA
<213> Homo sapiens

<400> 8
ggagcggtcc 10

<210> 9
<211> 8
<212> DNA
<213> Homo sapiens

<400> 9
ggcctgcc 8

<210> 10
<211> 53
<212> DNA
<213> Homo sapiens

<400> 10
ggccgcggtt ccctctcgat gcttcaggga accaggacag aaaggtcctt ccg 53

<210> 11
<211> 10
<212> DNA
<213> Homo sapiens

<400> 11
ggagcggtcc 10

<210> 12
<211> 53
<212> DNA
<213> Homo sapiens

<400> 12
ggccgcggtt ccctctcgat gcttcaggga accaggacag aaaggtcctt ccg 53

<210> 13
<211> 47
<212> DNA
<213> Homo sapiens

<400> 13
acacatataa catttacagg ggctcaaact gccgctgcct cctctcc 47

<210> 14
<211> 8
<212> DNA
<213> Homo sapiens

<400> 14
ggc tgcc 8

<210> 15
<211> 99
<212> DNA
<213> Homo sapiens

<400> 15
gggctcaaac tgccgctgcc tcctctccgg agcggtccgg cctgccggcc gcggttccct 60
ctcgatgctt cagggaacca ggacagaaag gtccttccg 99

<210> 16
<211> 122
<212> DNA
<213> Homo sapiens

<400> 16
acacatataa catttacagg ggctcaaact gccgctgcct cctctccgga gcggtccggc 60
ctgccggccg cggttccctc tcgatgcttc agggaaccag gacagaaagg tccttccgct 120
ag 122

## Description

The human genome is estimated to contain 50 x $10^6$ CpG dinucleotides, the predominant sequence recognition motif for mammalian DNA methyltransferases. Clusters of CpGs, or "CpG islands", are present in the promoter or intronic regions of approximately 40% of mammalian genes (Larsen et al., Genomics (1992) 13:1095-1107). Methylation of cytosine residues contained within CpG islands (i.e. "CpG island methylation") has generally been correlated with reduced gene expression, and is thought to play a fundamental role in many mammalian processes, including embryonic development, X-inactivation, genomic imprinting, regulation of gene expression, and host defense against parasitic sequences, as well as abnormal processes such as carcinogenesis, fragile site expression, and cytosine to thymine transition mutations. In addition alterations in methylation levels of CpGs occur under different physiologic and pathologic conditions. Accordingly, CpG methylation is an area of intense interest to the scientific community.

Given the number of CpGs and their association with CpG islands in the human genome, there is a great need for reliable, straightforward and high-throughput tools for their analysis. However, although several methodologies have been developed to study the methylation status of CpG dinucleotides, these methodologies generally fail to meet this need.

One conventional method for determining the methylation status of CpG dinucleotides involves bisulfite nucleotide sequencing. This method, developed by Frommer and colleagues (Proc. Natl. Acad. Sci. (1992) 89: 1827-1831), relies on the ability of sodium bisulfite to deaminate non-methylated cytosine residues into uracil in genomic DNA. In contrast methylated cytosine residues are resistant to this modification. After bisulfite treatment, target DNA is cloned and sequenced and the methylation status of individual CpG sites is then analyzed by comparing the obtained sequence with the sequence of the same DNA that has not been treated with bisulfite. Using this conventional bisulphite modification method, many investigators have addressed the importance of promoter CpG hypermethylation in the regulation of specific gene transcription in cancer (e.g., Hiltunen et al. 1997; Stirzaker et al. 1997; Rice et al. 1998; Melki et al. 1999). However, this method requires cloning and sequencing of individual DNA targets, and, as such, is labor intensive and is therefore generally restricted to the evaluation of DNA methylation on a gene-by-gene basis. Furtherr, because these methods are dependent on the complete chemical conversion of any umethylated CpGs in a sample, false positive results (e.g. unconverted non-methylated CpGs) are often obtained.

An alternative bisulphate modification assay for the methylation status of CpGs relies on sets of PCR primers that, although designed for the same target DNA, are specific to either the converted (i.e. unmethylated Cs changed to Ts) or unconverted (i.e. methylated Cs remain Cs) nucleotides in a bisulfite treated sample (Herman et al., 1996). The presence of methylation in a region of interest is detected by the presence of PCR products with the set of primers that are specific for unconverted sequences. Although less labor-intensive, this method is limited to assaying the methylation status of CpGs that are present in the recognition sites of the PCR primers, typically 20 to 30 nucleotides. Furthermore this method is also susceptible to false positives due to incomplete bisulfite conversion chemical reactions.

Many other conventional methods rely on restriction enzyme-based technologies. In these methods, a methylation-sensitive restriction endonuclease and a methylation-insensitive isoschizomer of that endonuclease are used to differentiate between methylated and unmethylated cytosines in the recognition motif for the endonucleases. In these methods, the methylation status of a particular CpG island is generally assessed by determining whether the CpG island is cleaved by a methylation sensitive enzyme that recognizes a methylated cytosine-containing motif within the CpG island. Typically, separate aliquots of the same genomic DNA are digested with each of the enzymes, and the methylation status of a CpG island in the DNA is deduced by detecting the presence or absence of specific DNA restriction fragments. In some methods, Southern blotting is used, which involves separating the digested DNA fragments on the basis of size (e.g., by gel electrophoresis), and hybridization with a labeled probe that detects the DNA fragments of interest. In other methods, a post-digest PCR amplification step is performed where a set oligonucleotide primers, one on each side of the methylation sensitive restriction site, is used to amplify the digested DNA. If the methylation sensitive enzyme does not digest a CpG island because the CpG island is methylated, PCR amplification products will be detected. Again, these methods are limited because they can only be designed for CpGs that occur within restriction sites, and they typically require detection of single DNA fragment using hybridization or PCR amplification, and, as such, are impractical as a high-throughput tool for investigating CpG island methylation. Further, amplification steps such as PCR amplification can bias certain sequences, leading to unreliable results.

Further techniques, such as differential methylation hybridization (DMH) (Huang et al., Human Mol. Genet. 8, 459-70, 1999); Not I-based differential methylation hybridization (see e.g., WO 02/086163 Al); restriction landmark genomic scanning (RLGS) (Plass et al., Genomics 58:254-62, 1999); methylation sensitive arbitrarily primed PCR (AP-PCR) (Gonzalgo et al., Cancer Res. 57: 594-599, 1997); and methylated CpG island amplification (MCA) (Toyota et. al., Cancer Res. 59: 2307-2312, 1999), have also been developed. However, these techniques are also unsuitable as high-throughput tools for investigating CpG methylation because they generally require a number of amplification steps or chemical treatments that lead to unreliable results.

Accordingly, while several methods have proved successful in assessing methylation of particular CpG islands, such methods are generally laborious or error prone and unsuitable for high-throughput studies of CpG island methylation.

As such, a great need still exists for reliable, straightforward and high-throughput methods for analysis of CpG island methylation. This invention meets this, and other needs.

## Relevant Literature

Literature of interest includes: Huang et al., (Human Mol. Genet. (1999) 8: 459-70), Plass et al., (Genomics (1999) 58:254-62), Gonzalgo et al., (Cancer Res. (1997) 57: 594-599), Toyota et. al., (Cancer Res. (1999) 59: 2307-2312), Cottrell et al, (Ann N Y Acad Sci. (2003) 983:120-130), Gitan et al., (Genome Research (2003) 12: 158-164), Kutyavin et al., (Nucl. Acids Res. (2002) 30: 4952-4959), Takai et al., (Proc. Natl. Acad. Sci. (2002) 99: 3740-3745); Strichman-Almashanu et al., (Genome Research (2002) 12:543-554); Sved et al., (Proc. Natl. Acad. Sci. (1990) 87:4692-6), Antequera et al., Proc. Natl. Acad. Sci. (1993) 90:11995-9 and Chen et al., (Am. J. Pathol. (2003) 163:37-45); published U.S. Patent Applications 20030211474, 20030215842, 20030186250, 20020123053, 20030129602 and 20020006623; and PCT publication WO 02/086163.

Methods and compositions for assessing CpG island methylation are provided. Specifically, the invention provides an unstructured nucleic acid (UNA) oligonucleotide that base pairs with, i.e., hybridizes to, CpG islands. The subject oligonucleotide may be present in an array, and find use in methods for evaluating methylation of CpG islands in cells. In one embodiment of the subject methods, a sample containing a CpG island is contacted with a methylation-sensitive restriction enzyme to produce a target composition, and binding of the target composition to a subject oligonucleotide is assessed. The subject compositions and methods may be used to compare CpG methylation patterns in cells, and, as such, may be employed in a variety of diagnostic and research applications. Kits and computer programming for use in practicing the subject methods are also provided.

Fig. 1 shows the chemical structures of several UNA nucleotides that find use in the subject methods.

Fig. 2 is a schematic representation of an embodiment of the subject invention.

Fig. 3 is a schematic representation of another embodiment of the subject invention.

Fig. 4A presents exemplary hypothetical results obtained from analysis of the human *Asparagine Synthetase* gene that is methylated at a CpG island, using methods of the invention. From top to bottom, the nucleic acid sequences shown in Fig. 4A are listed in the Sequence Listing as follows: SEQ ID NO:1, SEQ ID NO:2, SEQ ID NO:3, SEQ ID NO:4, and SEQ ID NO:5.

Fig. 4B presents exemplary hypothetical results obtained from analysis of the human *Asparagine Synthetase* gene that is unmethylated, using methods of the invention. The nucleic acid sequences in Fig. 4A are listed in the Sequence Listing as follows: SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15 and SEQ ID NO:16.

The term "nucleic acid" and "polynucleotide" are used interchangeably herein to describe a polymer of any length, e.g., greater than about 10 bases, greater than about 100 bases, greater than about 500 bases, greater than 1000 bases, usually up to about 10,000 or more bases composed of nucleotides, e.g., deoxyribonucleotides or ribonucleotides, or compounds produced synthetically (e.g., PNA as described in U.S. Patent No. 5,948,902 and the references cited therein) which can hybridize with naturally occurring nucleic acids in a sequence specific manner analogous to that of two naturally occurring nucleic acids, e.g., can participate in Watson-Crick base pairing interactions. Naturally-occurring nucleotides include guanine, cytosine, adenine and thymine (G, C, A and T, respectively).

An "unstructured nucleic acid" or "UNA" for short, as will be described in much greater detail below, is a nucleic acid containing non-natural nucleotides that bind to each other with reduced stability. For example, an unstructured nucleic acid may contain a G' residue and a C' residue, where these residues correspond to non-naturally occurring forms, i.e., analogs, of G and C that base pair with each other with reduced stability, but retain an ability to base pair with naturally occurring C and G residues, respectively.

The terms "ribonucleic acid" and "RNA" as used herein mean a polymer composed of ribonucleotides.

The terms "deoxyribonucleic acid" and "DNA" as used herein mean a polymer composed of deoxyribonucleotides.

The term "oligonucleotide" as used herein denotes single stranded nucleotide multimers of from about 10 to 100 nucleotides and up to 200 nucleotides in length. Oligonucleotides are usually synthetic and, in many embodiments, are under 50 nucleotides in length.

The term "oligomer" is used herein to indicate a chemical entity that contains a plurality of monomers. As used herein, the terms "oligomer" and "polymer" are used interchangeably, as it is generally, although not necessarily, smaller "polymers" that are prepared using the functionalized substrates of the invention, particularly in conjunction with combinatorial chemistry techniques. Examples of oligomers and polymers include polydeoxyribonucleotides (DNA), polyribonucleotides (RNA), other nucleic acids that are C-glycosides of a purine or pyrimidine base, polypeptides (proteins), polysaccharides (starches, or polysugars), and other chemical entities that contain repeating units of like chemical structure.

The term "sample" as used herein relates to a material or mixture of materials, typically, although not necessarily, in fluid form, containing one or more components of interest.

The terms "nucleoside" and "nucleotide" are intended to include those moieties that contain not only the known purine and pyrimidine bases, but also other heterocyclic bases that have been modified. Such modifications include methylated purines or pyrimidines, acylated purines or pyrimidines, alkylated riboses or other heterocycles. In addition, the terms "nucleoside" and "nucleotide" include those moieties that contain not only conventional ribose and deoxyribose sugars, but other sugars as well. Modified nucleosides or nucleotides also include modifications on the sugar moiety, e.g., wherein one or more of the hydroxyl groups are replaced with halogen atoms or aliphatic groups, or are functionalized as ethers, amines, or the like.

The phrase "surface-bound polynucleotide" refers to a polynucleotide that is immobilized on a surface of a solid substrate, where the substrate can have a variety of configurations, e.g., a sheet, bead, or other structure. In certain embodiments, the collections of CpG UNA oligonucleotides employed herein are present on a surface of the same planar support, e.g., in the form of an array.

The phrase "labeled population of nucleic acids" refers to mixture of nucleic acids that are detectably labeled, e.g., fluorescently labeled, such that the presence of the nucleic acids can be detected by assessing the presence ofthe label. A labeled population of nucleic acids is "made from" a "CpG island composition" or a "sample composition", the composition is usually employed as template for making the population of nucleic acids.

The term "array" encompasses the term "microarray" and refers to an ordered array presented for binding to nucleic acids and the like.

An "array," includes any two-dimensional or substantially two-dimensional (as well as a three-dimensional) arrangement of spatially addressable regions bearing nucleic acids, particularly oligonucleotides or synthetic mimeties thereof, and the like, e.g., UNA oligonucleotides. Where the arrays are arrays of nucleic acids, the nucleic acids may be adsorbed, physisorbed, chemisorbed, or covalently attached to the arrays at any point or points along the nucleic acid chain.

Any given substrate may carry one, two, four or more arrays disposed on a surface of the substrate. Depending upon the use, any or all of the arrays may be the same or different from one another and each may contain multiple spots or features. A typical array may contain one or more, including more than two, more than ten, more than one hundred, more than one thousand, more ten thousand features, or even more than one hundred thousand features, in an area of less than 20 $cm^2$ or even less than 10 $cm^2$, e.g., less than about 5$cm^2$, including less than about 1 $cm^2$, less than about 1 $mm^2$, e.g., 100 $\mu m^2$, or even smaller. For example, features may have widths (that is, diameter, for a round spot) in the range from a 10 μm to 1.0 cm. In other embodiments each feature may have a width in the range of 1.0 μm to 1.0 mm, usually 5.0 μm to 500 μm, and more usually 10 μm to 200 μm. Non-round features may have area ranges equivalent to that of circular features with the foregoing width (diameter) ranges. At least some, or all, of the features are of different compositions (for example, when any repeats of each feature composition are excluded the remaining features may account for at least 5%, 10%, 20%, 50%, 95%, 99% or 100% of the total number of features). Inter-feature areas will typically (but not essentially) be present which do not carry any nucleic acids (or other biopolymer or chemical moiety of a type of which the features are composed). Such inter-feature areas typically will be present where the arrays are formed by processes involving drop deposition of reagents but may not be present when, for example, photolithographic array fabrication processes are used. It will be appreciated though, that the inter-feature areas, when present, could be of various sizes and configurations.

Each array may cover an area of less than 200 $cm^2$, or even less than 50 $cm^2$, 5 $cm^2$, 1 $cm^2$, 0.5 $cm^2$, or 0.1 $cm^2$. In certain embodiments, the substrate carrying the one or more arrays will be shaped generally as a rectangular solid (although other shapes are possible), having a length of more than 4 mm and less than 150 mm, usually more than 4 mm and less than 80 mm, more usually less than 20 mm; a width of more than 4 mm and less than 150 mm, usually less than 80 mm and more usually less than 20 mm; and a thickness of more than 0.01 mm and less than 5.0 mm, usually more than 0.1 mm and less than 2 mm and more usually more than 0.2 and less than 1.5 mm, such as more than about 0.8 mm and less than about 1.2 mm. With arrays that are read by detecting fluorescence, the substrate may be of a material that emits low fluorescence upon illumination with the excitation light. Additionally in this situation, the substrate may be relatively transparent to reduce the absorption of the incident illuminating laser light and subsequent heating if the focused laser beam travels too slowly over a region. For example, the substrate may transmit at least 20%, or 50% (or even at least 70%, 90%, or 95%), of the illuminating light incident on the front as may be measured across the entire integrated spectrum of such illuminating light or alternatively at 532 nm or 633 nm.

Arrays can be fabricated using drop deposition from pulse-jets of either precursor units (such as nucleotide or amino acid monomers) in the case *of in situ* fabrication, or the previously obtained nucleic acid. Such methods are described in detail in, for example, the previously cited references including US 6,242,266, US 6,232,072, US 6,180,351, US 6,171,797, US 6,323,043, U.S. Patent Application Serial No. 09/302,898 filed April 30, 1999 by Caren et al., and the references cited therein. As already mentioned, these references are incorporated herein by reference. Other drop deposition methods can be used for fabrication, as previously described herein. Also, instead of drop deposition methods, photolithographic array fabrication methods may be used. Inter-feature areas need not be present particularly when the arrays are made by photolithographic methods as described in those patents.

An array is "addressable" when it has multiple regions of different moieties (e.g., different oligonucleotide sequences) such that a region (i.e., a "feature" or "spot" of the array) at a particular predetermined location (i.e., an "address") on the array will detect a particular sequence. Array features are typically, but need not be, separated by intervening spaces. In the case of an array in the context of the present application, the "population of labeled nucleic acids" or "sample composition" and the like will be referenced as a moiety in a mobile phase (typically fluid), to be detected by "surface-bound polynucleotides" which are bound to the substrate at the various regions. These phrases are synonymous with the arbitrary terms "target" and "probe", or "probe" and "target", respectively, as they are used in other publications.

A "scan region" refers to a contiguous (preferably, rectangular) area in which the array spots or features of interest, as defined above, are found or detected. Where fluorescent labels are employed, the scan region is that portion of the total area illuminated from which the resulting fluorescence is detected and recorded. Where other detection protocols are employed, the scan region is that portion of the total area queried from which resulting signal is detected and recorded. For the purposes of this invention and with respect to fluorescent detection embodiments, the scan region includes the entire area of the slide scanned in each pass of the lens, between the first feature of interest, and the last feature of interest, even if there exist intervening areas that lack features of interest.

An "array layout" refers to one or more characteristics of the features, such as feature positioning on the substrate, one or more feature dimensions, and an indication of a moiety at a given location. "Hybridizing" and "binding", with respect to nucleic acids, are used interchangeably.

The term "stringent assay conditions" as used herein refers to conditions that are compatible to produce binding pairs of nucleic acids, e.g., probes and targets, of sufficient complementarity to provide for the desired level of specificity in the assay while being incompatible to the formation of binding pairs between binding members of insufficient complementarity to provide for the desired specificity. The term stringent assay conditions refers to the combination of hybridization and wash conditions.

A "stringent hybridization" and "stringent hybridization wash conditions" in the context of nucleic acid hybridization (e.g., as in array, Southern or Northern hybridizations) are sequence dependent, and are different under different experimental parameters. Stringent hybridization conditions that can be used to identify nucleic acids within the scope of the invention can include, e.g., hybridization in a buffer comprising 50% formamide, 5×SSC, and 1% SDS at 42°C, or hybridization in a buffer comprising 5×SSC and 1% SDS at 65°C, both with a wash of 0.2xSSC and 0.1% SDS at 65°C. Exemplary stringent hybridization conditions can also include a hybridization in a buffer of 40% formamide, 1 M NaCl, and 1% SDS at 37°C, and a wash in 1×SSC at 45°C. Alternatively, hybridization to filter-bound DNA in 0.5 M $NaHPO_4$, 7% sodium dodecyl sulfate (SDS), 1 mnM EDTA at 65°C, and washing in 0.1×SSC/0.1% SDS at 68°C can be employed. Yet additional stringent hybridization conditions include hybridization at 60°C or higher and 3 × SSC (450 mM sodium chloride/45 mM sodium citrate) or incubation at 42°C in a solution containing 30% formamide, 1 M NaCl, 0.5% sodium sarcosine, 50 mM MES, pH 6.5. Those of ordinary skill will readily recognize that alternative but comparable hybridization and wash conditions can be utilized to provide conditions of simitar stringency.

In certain embodiments, the stringency of the wash conditions determines whether a nucleic acid is specifically hybridized to a probe. Wash conditions used to identify nucleic acids may include, e.g.: a salt concentration of about 0.02 molar at pH 7 and a temperature of at least about 50 °C or about 55°C to about 60°C; or, a salt concentration of about 0.15 M NaCl at 72°C for about 15 minutes; or, a salt concentration of about 0.2xSSC at a temperature of at least about 50°C or about 55 °C to about 60°C for about 15 to about 20 minutes; or, the hybridization complex is washed twice with a solution with a salt concentration of about 2xSSC containing 0.1 % SDS at room temperature for 15 minutes and then washed twice by 0.1×SSC containing 0.1% SDS at 68°C for 15 minutes; or, equivalent conditions. Stringent conditions for washing can also be, e.g., 0.2xSSC/0.1% SDS at 42°C. In instances wherein the nucleic acid molecules are deoxyoligonucleotides ("oligos"), stringent conditions can include washing in 6×SSC/0.05% sodium pyrophosphate at 37 °C (for 14-base oligos), 48 °C (for 17-base oligos), 55°C (for 20-base oligos), and 60°C (for 23-base oligos). See Sambrook, Ausubel, or Tijssen (cited below) for detailed descriptions of equilvalent hybridization and wash conditions and for reagents and buffers, e.g., SSC buffers and equivalent reagents and conditions.

A specific example of stringent assay conditions is rotating hybridization at 65°C in a salt based hybridization buffer with a total monovalent cation concentration of 1.5M (e.g., as described in U.S. Patent Application No. 09/655,482 filed on September 5, 2000, the disclosure of which is herein incorporated by reference) followed by washes of 0.5X SSC and 0.1X SSC at room temperature.

Stringent hybridization conditions may also include a "prehybridization" of aqueous phase nucleic acids with complexity-reducing nucleic acids to suppress repetitive sequences and reduce the complexity of the sample prior to hybridization. For example, certain stringent hybridization conditions include, prior to any hybridization to surface-bound polynucleotides, hybridization with Cot-1 DNA, or the like.

Stringent assay conditions are hybridization conditions that are at least as stringent as the above representative conditions, where a given set of conditions are considered to be at least as stringent if substantially no additional binding complexes that lack sufficient complementarity to provide for the desired specificity are produced in the given set of conditions as compared to the above specific conditions, where by "substantially no more" is meant less than about 5-fold more, typically less than about 3-fold more. Other stringent hybridization conditions are known in the art and may also be employed, as appropriate.

The term "mixture", as used herein, refers to a combination of elements, that are interspersed and not in any particular order. A mixture is heterogeneous and not spatially separable into its different constituents. Examples of mixtures of elements include a number of different elements that are dissolved in the same aqueous solution, or a number of different elements attached to a solid support at random or in no particular order in which the different elements are not specially distinct. In other words, a mixture is not addressable. To be specific, an array of surface-bound polynucleotides, as is commonly known in the art and described below, is not a mixture of surface-bound polynucleotides because the species of surface-bound polynucleotides are spatially distinct and the array is addressable.

"Isolated" or "purified" generally refers to isolation of a substance (compound, polynucleotide, protein, polypeptide, polypeptide composition) such that the substance comprises a significant percent (e.g., greater than 2%, greater than 5%, greater than 10%, greater than 20%, greater than 50%, or more, usually up to about 90%-100%) of the sample in which it resides. In certain embodiments, a substantially purified component comprises at least 50%, 80%-85%, or 90-95% of the sample. Techniques for purifying polynucleotides and polypeptides of interest are well-known in the art and include, for example, ion-exchange chromatography, affinity chromatography and sedimentation according to density. Generally, a substance is purified when it exists in a sample in an amount, relative to other components of the sample, that is not found naturally.

The terms "determining", "measuring", "evaluating", "assessing" and "assaying" are used interchangeably herein to refer to any form of measurement, and include determining if an element is present or not. These terms include both quantitative and/or qualitative determinations. Assessing may be relative or absolute. "Assessing the presence of" includes determining the amount of something present, as well as determining whether it is present or absent.

The term "using" has its conventional meaning, and, as such, means employing, e.g., putting into service, a method or composition to attain an end. For example, if a program is used to create a file, a program is executed to make a file, the file usually being the output of the program. In another example, if a computer file is used, it is usually accessed, read, and the infonnation stored in the file employed to attain an end. Similarly if a unique identifier, e.g., a barcode is used, the unique identifier is usually read to identify, for example, an object or file associated with the unique identifier.

If a subject CpG oligonucleotide "corresponds to" or is "for" a certain CpG island, the oligonucleotide usually base pairs with, i.e., specifically hybridizes to, that CpG island. As will be discussed in greater detail below, a CpG oligonucleotide for a particular CpG island and the particular CpG island, or complement thereof, usually contain at least one region of contiguous nucleotides that is identical in sequence (with the exception of any modified nucleotides).

Methods and compositions for assessing CpG island methylation are provided. Specifically, the invention provides an unstructured nucleic acid (UNA) oligonucleotide that base pairs with, i.e., hybridizes to, CpG islands. The subject oligonucleotide may be present in an array, and find use in methods for evaluating methylation of CpG islands in cells. In one embodiment of the subject methods, a sample containing a CpG island is contacted with a methylation-sensitive restriction enzyme to produce a target composition, and binding of the target composition to a subject oligonucleotide is assessed. The subject compositions and methods may be used to compare CpG methylation patterns in cells, and, as such, may be employed in a variety of diagnostic and research applications. Kits and computer programming for use in practicing the subject methods are also provided.

Before the subject invention is described further, it is to be understood that the invention is not limited to the particular embodiments of the invention described below, as variations of the particular embodiments may be made and still fall within the scope of the appended claims. It is also to be understood that the terminology employed is for the purpose of describing particular embodiments, and is not intended to be limiting. Instead, the scope of the present invention will be established by the appended claims.

In this specification and the appended claims, the singular forms "a," "an" and "the" include plural reference unless the context clearly dictates otherwise. Unless defined otherwise, all technical and scientific terms used herein have the same meaning as commonly understood to one of ordinary skill in the art to which this invention belongs.

Where a range of values is provided, it is understood that each intervening value, to the tenth of the unit of the lower limit unless the context clearly dictates otherwise, between the upper and lower limit of that range, and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood to one of ordinary skill in the art to which this invention belongs. Although any methods, devices and materials similar or equivalent to those described herein can be used in the practice or testing of the invention, the preferred methods, devices and materials are now described.

All publications mentioned herein are incorporated herein by reference for the purpose of describing and disclosing the invention components that are described in the publications that might be used in connection with the presently described invention.

As summarized above, the present invention provides methods and compositions for assessing methylation of a CpG island. With reference to Fig. 2, showing an exemplary embodiment of the invention, the methods usually involve contacting a CpG island with a methylation-sensitive enzyme to produce a target composition, contacting, i.e., hybridizing, a labeled target composition with an array containing an CpG unstructured nucleic acid oligonucleotide feature, and assessing binding of the labeled target composition to the CpG unstructured nucleic acid oligonucleotide feature.

In further describing the present invention, CpG unstructured nucleic acid oligonucleotide and arrays thereof will be described first, followed by a detailed description of how the subject oligonucleotides may be used to assess CpG methylation. Finally, representative kits and computer programming for use in practicing the subject methods will be discussed.

## ***CpG UNSTRUCTURED NUCLEIC ACID OLIGONUCLEOTIDES***

As mentioned above, the invention provides a CpG unstructured nucleic acid (UNA) oligonucleotide. By "CpG unstructured nucleic acid oligonucleotide" or "CpG UNA oligonucleotide", for short, is meant an oligonucleotide that a) contains at least one UNA nucleotide and therefore has reduced secondary structure, and, b) corresponds to, i.e., has a sequence that is at least partially complementary to or the same as and will base-pair with, a CpG island.

"Unstructured nucleic acid", as used herein, refers to a nucleic acid molecule containing at least one or usually at least one pair of non-natural nucleotides (i.e., A', G', C' or T'; or A' and T' or C' and G') that exhibits reduced levels of secondary structure, as compared to a nucleic acid molecule of the same nucleotide sequence containing only naturally-occurring nucleotides (A, G, C and T). UNAs maintain an ability to hybridize to a nucleic acid that has a sequence of naturally occurring nucleotides that is complementary to the UNA sequence.

In certain embodiments, UNAs have a reduced ability to form secondary structure because of their reduced ability to form intramolecular hydrogen bond base pairs. In these embodiments, one or both of the nucleotides that together form at least one complementary base pair (e.g., one or more G and/or C residues), is substituted with a nucleotide analog so that the base pair is no longer formed, or is formed at a reduced level. In some embodiments, at least one hydrogen bond is maintained in a modified base pair (e.g. an A'/T' base pair), however, in certain modified base pairs, (e.g., a C'/G' base pair) up to two hydrogen bonds may be maintained.

In certain embodiments, the nucleotide analogs 2-aminoadenosine, 2-thiothymidine, inosine (I), and pyrrolo-pyrimidine (P) are used to produce LTNAs that are unable to form stable intra-molecular base pairs, yet retain their ability to form Watson-Crick base pairs with the four natural nucleotides. 2-aminoadenosine and 2-thiothymidine, for example, are unable to base pair together but are capable of base pairing with natural thymidine and natural adenine, respectively. Further, inosine and pyrroloyrimidine are unable to base pair together but are capable of binding with natural cystosine and guanine, respectively. Fig. 3 shows various exemplary UNA nucleotides base pairing with other UNA and natural nucleotides, wherein "X" denotes a base pair with low stability.

The subject unstructured nucleic acid oligonucleotides are, accordingly, UNAs that are about 10 to about 200 bases in length. In certain embodiments, however, the subject oligonucleotides may be about 10 to about 100 bases, about 20 to about 80 bases, about 30 to about 60 bases, or about 40 to about 50 bases in length. In other embodiments, the subject UNA oligonucleotides are about 50-70 bases and usually approximately 60 bases in length.

The subject oligonucleotides may contain both UNA nucleotides and naturally occurring nucleotides, or may be entirely made up of UNA nucleotides. However, since, as will be discussed below, the subject UNA oligonucleotides are typically "G/C rich", i.e., contain greater than about 50% G or C residues, most of the subject oligonucleotides contain G and/or C UNA nucleotide analogs, e.g., inosine and/or pyrroloyrimidine, or derivatives thereof, as discussed above. Accordingly, the subject oligonucleotides may contain 1 or more, 2 or more, about 4 or more, about 6 or more, about 8 or more, about 10 or more, about 12 or more, about 16 or more or about 20 or more, usually up to about 24 or 30 or more, UNA nucleotides or base-pairing pairs of UNA nucleotides.

UNAs are generally known in the art and are described in Published U.S. Patent Application No. 20030211474, which is incorporated by reference in its entirety, and Kutyavin et al., (Nucl. Acids. Res. (2002) 30:4952-4959). Further details of UNAs may be found in U.S. Patent Application Serial Number 10/324,409, filed December 18, 2002, which is also incorporated by reference in its entirety. As detailed therein, UNAs may be made enzymatically or synthetically.

As noted above, the subject oligonucleotides base pair with "CpG islands", where a CpG island is defined herein as any discrete region of a genome that contains a CpG that is, or is predicted to be, a target for a cellular methyltransferase. CpG islands may be high-density CpG islands, such as those defined by Gardiner-Garden and Frommer (J. Mol. Biol. (1987) 196:261-82), i.e., any stretch of DNA that is at least 200bp in length that has a C + G content of at least 50% and an observed CpG/expected CpG ratio of greater than or equal to 0.60. CpG islands may also be low-density CpG islands, containing CpG dinucleotides that occur at a lower density in a given region. The methylation status of these low density CpG islands varies under different physiologic and pathologic conditions, including ageing and cancer, Toyota and Issa, (Seminars in Cancer Biology (1999) 9:349-357). In general, CpG islands are generally found proximal to (i.e., within 1 kb, 3 kb, or about 5 kb of) the transcriptional start sites of eukaryotic genes. It has been estimated that there are approximately 45,000 CpG islands in the human genome and 37,000 CpG islands in the mouse genome (Antequera et al., Proc. Natl. Acad. Sci. (1993) 90:11995-9).

A detailed discussion of CpG islands, methods for their identification, and many examples of CpG islands in human chromosomes is found in a variety of publications, including: Larsen et al., (Genomics (1992), 13:1095-1107), Takai et al., (Proc. Natl. Acad. Sci. (2002) 99:3740-3745), Antequera et al., (Proc. Natl. Acad. Sci. (1993) 90:11995-9) and Ioshikhes et al., (Nat Genet. (2000) 26:61-3). Accordingly, CpG islands are well known in the art and need not be described herein in any more detail.

A CpG oligonucleotide is an oligonucleotide that corresponds to, i.e., hybridizes to and may be used to detect, a particular CpG island. In most embodiments, such an oligonucleotide is specific for a particular CpG island, i.e., is "CpG island-specific", in that it can detect a single CpG island, even in the presence of other chromosomal fragments (e.g., other CpG islands). In other words, a subject oligonucleotide contains a nucleic acid sequence that is present in or complementary to a single CpG island. An oligonucleotide that merely contains a CpG dinucleotide cannot be a CpG oligonucleotide unless that oligonucleotide, and the CpG dinucleotide contained therein, corresponds to region of a genome that is, or predicted to be, a site of CpG methylation. In other words, an oligonucleotide that contains a CpG dinucleotide that does not correspond to a site of genomic methylation is not a CpG oligonucleotide, under the present definitions.

In certain embodiments, as will be discussed in greater detail below, the subject oligonucleotides may bind to an uncleaved, i.e., intact, CpG island, but not bind under high stringency hybridization conditions to a CpG island that is cleaved by a methylation-sensitive enzyme. In these embodiments, a subject oligonucleotide may also contain a sequence that corresponds to the recognition sequence for a methylation-sensitive enzyme. In other words, if a methylation-sensitive enzyme cleaves at a site containing the contiguous nucleotides "$CC^mGG$" (where $C^m$ is methylcytosine), a subject oligonucleotide may also contain that sequence. In particular embodiments, the enzyme cleavage site corresponds to a site proximal to (i.e., is at or within 1, 2, 3, 4, 5, 6, 7, 8 about 10, about 12, about 15 or about 20 nucleotides) the middle of the oligonucleotide. In other words, the site corresponding to the cleavage site of a methylation-sensitive enzyme for an oligonucleotide of size N, is usually found at position 0.5N+/- 1, 2, 3, 4, etc., usually up to about 20.

In many embodiments, the subject oligonucleotides have been designed according to one or more particular parameters to be suitable for use in a given application, where representative parameter include, but are not limited to: length, melting temperature ($T_m$), non-homology with other regions of the genome, signal intensities, kinetic properties under hybridization conditions, etc., see e.g., U.S. Patent No. 6,251,588, the disclosure of which is herein incorporated by reference. In certain embodiments, the entire length of the subject oligonucleotides is employed in hybridizing to particular CpG island, while in other embodiments, only a portion of the subject oligonucleotide has sequence that hybridizes a CpG island, e.g., where a portion of the oligonucleotide serves as a tether. For example, a given oligonucleotide may include a 30 nt long CpG -specific sequence linked to a 30 nt tether, such that the oligonucleotide is a 60-mer of which only a portion, e.g., 30 nt long, is CpG island-specific.

## ***ARRAY PLATFORMS***

In certain embodiments of the invention the CpG UNA oligonucleotides are "surface-bound CpG UNA oligonucleotides", where such an oligonucleotide is a CpG UNA oligonucleotide that is bound, usually covalently but in certain embodiments non-covalently, to a surface of a solid substrate, i.e., a sheet, bead, or other structure. In certain embodiments, surface-bound UNA oligonucleotides may be immobilized on a surface of a planar support, e.g., as part of an array.

A "CpG UNA oligonucleotide feature" is a feature of an array, i.e., a spatially addressable area of an array, as described above, that contains a plurality of surface-bound CpG UNA oligonucleotides. Accordingly, a feature contains "surface-bound" oligonucleotides that are bound, usually covalently, to an area of an array. In most embodiments a single type of oligonucleotide is present in each CpG UNA oligonucleotide feature (i.e., all the oligonucleotides in the feature have the same sequence). However, in certain embodiments, the oligonucleotides in a feature may be a mixture of oligonucleotides with different sequence.

The subject arrays may contain a single CpG UNA oligonucleotide feature. However, in many embodiments, the subject arrays may contain more than one such feature, and those features may correspond to (i.e., may be used to detect) a plurality of CpG islands of a genome. Accordingly, the subject arrays may contain a plurality of features (i. e., 2 or more, about 5 or more, about 10 or more, about 15 or more, about 20 or more, about 30 or more, about 50 or more, about 100 or more, about 200 or more, about 500 or more, about 1000 or more, usually up to about 10,000 or about 20,000 or more features, etc.), each containing a different CpG UNA oligonucleotide. In certain embodiments, therefore, the subject arrays contain a plurality of subject oligonucleotide features that correspond to a plurality of CpG islands of a genome. In particular embodiments, therefore, the subject arrays may contain CpG UNA oligonucleotide features for, i.e., corresponding to, all of the predicted CpG islands of a particular genome. The subject arrays for investigating methylation status of human CpG islands may therefore contain at least up to 45,000 different CpG UNA oligonucleotide features.

The subject CpG UNA oligonucleotide features are usually present in an array of oligonucleotide features. In general, arrays suitable for use in performing the subject methods contain a plurality (i.e., at least about 100, at least about 500, at least about 1000, at least about 2000, at least about 5000, at least about 10,000, at least about 20,000, usually up to about 100,000 or more) of addressable features containing oligonucleotides that are linked to a usually planar solid support. Features on a subject array usually contain polynucleotides that hybridize to, i.e., bind to, genomic sequences from a cell. Accordingly, "CpG island methylation arrays", typically involve an array containing a plurality of different CpG UNA oligonucleotides that are addressably arrayed. In certain embodiments, the subject array features may also contain other polynucleotides, such as other oligonucleotides, or other cDNAs, or inserts from phage BACs or plasmids clones. As such, while the subject genome CpG island methylation arrays usually contain features of oligonucleotides, they may also contain features of polynucleotides that are about 201-5000 bases in length, about 5001-50,000 bases in length, or about 50,001-200,000 bases in length, depending on the platform used.

If other polynucleotide features are present on a subject array, they may be interspersed with, or in a separately-hybridizable part of the array from, the subject oligonucleotides.

In particular embodiments, CpG islands of interest are represented by at least 2, about 5, or about 10 or more, usually up to about 20 features containing oligonucleotides of different, non-overlapping, or, in some embodiments, overlapping, sequence.

In general, methods for the preparation of polynucleotide arrays are well known in the art (see, e.g., Harrington et al,. Curr Opin Microbiol. (2000) 3:285-91, and Lipshutz et al., Nat Genet. (1999) 21:20-4) and need not be described in any great detail. As is known, UNAs may be synthesized synthetically (Kutyavin et al., Nucl. Acids Res. (2002) 30: 4952-4959).

The subject CpG UNA oligonucleotide arrays can be fabricated using any means, including drop deposition from pulse jets or from fluid-filled tips, etc, or using photolithographic means. Either polynucleotide precursor units (such as nucleotide monomers), in the case of in situ fabrication, or previously synthesized polynucleotides (i.e., UNA oligonucleotides) can be deposited. Such methods are described in detail in, for example U.S. patents 6,242,266, 6,232,072, 6,180,351, 6,171,797, 6,323,043, etc., the disclosures of which are herein incorporated by reference.

## METHODS FOR EVALUATING METHYLATION OF A CpG ISLAND

The invention provides a method for evaluating methylation of a CpG island. In general, the method involves contacting a CpG island with a methylation-sensitive restriction enzyme to produce a target composition, and assessing binding of the target composition to a CpG L1NA oligonucleotide for that CpG island. In many embodiments, binding of the target composition to the CpG UNA oligonucleotide indicates that the CpG island is methylated, and lack of binding of the target composition indicates that the CpG island is not methylated (unmethylated). Accordingly, methylation of a CpG island may be assessed.

The first steps of this method are generally similar to conventional methods for assessing CpG island methylation in that a genomic sample containing a CpG island is usually provided. Methods for making such genomic samples are generally well known in the art and described in the prior art publications discussed in the background section herein, and, in well known laboratory manuals (e.g., Ausubel, et al., Short Protocols in Molecular Biology, 3rd ed., Wiley & Sons 1995 and Sambrook et al., Molecular Cloning: A Laboratory Manual, Third Edition, 2001 Cold Spring Harbor, N.Y. for example).

Once a genomic sample is prepared, it is usually separated into at least two or more equal parts (e.g., an equal volume of the sample is aliquoted into different vessels), and at least one of those parts is contacted with a methylation-sensitive enzyme that only cleaves at unmethylated recognition sites, under conditions suitable for activity of that enzyme. The restriction enzymes *Bst*UI, *Sma*I, *Sac*II, *Eag*I, *Msp*I, *Hpa*II, *Hha*I and *Bss*HII are methylation-sensitive enzymes that are suitable for use in the subject methods. These enzymes are purchasable from a variety of sources, e.g., Invitrogen (Carlsbad, CA) and Stratagene (La Jolla, CA), and conditions suitable for their activity are usually supplied with the enzyme when purchased. Accordingly, a genomic sample is contacted with a methylation-sensitive enzyme, and any unmethylated CpG islands in the genomic samples are cleaved at the recognition site for the enzyme. In many embodiments, the cleavage site of the enzyme encompasses a "CpG" dinucleotide, and the enzyme fails to cleave the CpG island if the CpG island is methylated. The product of the reaction is termed herein "target composition". Target compositions may contain cleaved CpG islands, uncleaved CpG islands, or a mixture thereof. In other words, if a sample contains a population of the same CpG island, none, some or all of these islands may be methylated. Accordingly, target compositions made by contacting that sample with a methylation-sensitive enzyme may contain CpG islands that are intact, cleaved, or a mixture thereof.

In certain embodiments, prior, during or after contacting the genomic extract with a methylation-sensitive enzyme, the genomic extract may optionally be contacted, under suitable conditions, with one or more restriction endonucleases that recognize cleavage sites that generally lie outside of CpG islands. This contacting step generally cleaves the DNA in the extract into fragments in which CpG islands, methylated or unmethylated, are intact. The restriction enzymes *Alu*I, *Rsa*I, *Mse*I, *Tsp*509I, *Nla*III and *Bfa*I, as well as many others, are enzymes that are suitable for this step of the subject methods, if employed. Again, these enzymes are purchasable from a variety of sources, e. g., Invitrogen (Carlsbad, CA) and Stratagene (La Jolla, CA), and conditions suitable for their activity are usually supplied with the enzyme when purchased.

The target composition is then usually labeled to make a population of labeled nucleic acids. In general, a target composition may be labeled using methods that are well known in the art (e.g., primer extension, random-priming, nick translation, etc.; see, e.g., Ausubel, et al., Short Protocols in Molecular Biology, 3rd ed., Wiley & Sons 1995 and Sambrook et al., Molecular Cloning: A Laboratory Manual, Third Edition, 2001 Cold Spring Harbor, N.Y.), and, accordingly, such methods do not need to be described here in great detail. In particular embodiments, the target composition is usually labeled with fluorescent label, which labels will be described in greater detail below.

After labeling, the target composition is contacted with a subject CpG UNA oligonucleotide under conditions of stringency, usually high stringency, and any binding of the target composition to the oligonucleotide is detected by detecting the label associated with the target composition. Since the subject LINAs usually do not bind to cleaved CpG islands, any binding of the target composition to the subject oligonucleotide indicates that that CpG island corresponding to the subject oligonucleotide is methylated.

In some embodiments, binding of the target composition is assessed with respect to binding of at least one control target composition. In general, suitable control target compositions are made from a second part of the genomic sample, as described above. Accordingly, in these embodiments, a genomic extract is prepared, divided into equal parts, and those equal parts are used to make a target composition and at least one target composition control. Target composition controls are usually identical to the target composition except that they are not contacted with the methylation-sensitive enzyme, or, in other embodiments, are contacted with a methylation-insensitive isoschizomer of the methylation-sensitive enzyme used to make the target composition. Suitable methylation-insensitive enzymes are well known in the art, and include, e.g. *Msp*I, a methylation-insensitive isoschizomer of *Hpa*II, and *Xma*I a methylation-insensitive isoschizomer of *Sma*I.

Accordingly, a target composition and a control target composition are usually prepared and labeled, and relative binding of the compositions to a subject CpG UNA oligonucleotide is assessed. Since the subject oligonucleotide is usually a surface-bound oligonucleotide that is present in a feature of an array, in many embodiments, the target compositions are labeled and contacted with at least one array containing a subject oligonucleotide feature, under high stringency conditions.

Accordingly, many embodiments of the subject methods involve labeling, e.g., distinguishably labeling, two target compositions to produce a first and second population of labeled nucleic acids, and assessing binding of the labeled nucleic acids to a subject feature, i.e., a CpG UNA oligonucleotide feature. In many embodiments, the methods generally follow the methods that are well known in the art and described in, e.g., Pinkel et al., (Nat. Genet. (1998) 20: 207-211); Hodgson et al., (Nat. Genet. (2001) 29:459-464); and Wilhelm et al., (Cancer Res. (2002) 62: 957-960), except that CpG island methylation may be assessed by evaluating binding to the subject feature.

In practicing the subject methods, the target compositions are labeled to provide at least two different populations of labeled nucleic acids that are to be compared. The populations of nucleic acids may be labeled with the same label or different labels, depending on the actual assay protocol employed. For example, where each population is to be contacted with different but identical arrays, each nucleic acid population may be labeled with the same label. Alternatively, where both populations are to be simultaneously contacted with a single array of surface-bound oligonucleotides, i.e., cohybridized, to the same array of immobilized nucleic acids, target compositions are generally distinguishably labeled with respect to each other.

The compositions are sometimes labeled using "distinguishable" labels in that the labels that can be independently detected and measured, even when the labels are mixed. In other words, the amounts of label present (e. g., the amount of fluorescence) for each of the labels are separately determinable, even when the labels are co-located (e.g., in the same tube or in the same duplex molecule or in the same feature of an array). Suitable distinguishable fluorescent label pairs useful in the subject methods include Cy-3 and Cy-5 (Amersham Inc., Piscataway, NJ), Quasar 570 and Quasar 670 (Biosearch Technology, Novato CA), Alexafluor555 and Alexafluor647 (Molecular Probes, Eugene, OR), BODIPY V-1002 and BODIPY V1005 (Molecular Probes, Eugene, OR), POPO-3 and TOTO-3 (Molecular Probes, Eugene, OR), fluorescein and Texas red (Dupont, Bostan MA) and POPRO3 and TOPRO3 (Molecular Probes, Eugene, OR). Further suitable distinguishable detectable labels may be described in Kricka et al. (Ann Clin Biochem. 39:114-29, 2002).

In many embodiments, the population of labeled nucleic acids does not have reduced (i.e., has non-reduced) complexity, as compared to the initial genomic sample. A non-reduced complexity collection is one that is not produced in a manner designed to reduce the complexity of the sample. A product composition is considered to be a non-reduced complexity product composition as compared to the initial nucleic acid source from which it is prepared if there is a high probability that a sequence of specific length randomly chosen from the sequence of the initial genomic source is present in the product composition, either in a single nucleic acid member of the product or in a "concatamer" of two different nucleic acid members of the product (i.e., in a virtual molecule produced by joining two different members to produce a single molecule). In other words, if there is a high probably that an N-mer sequence (i.e., a sequence of "N" nucleotides) that is randomly chosen from the initial source has the same sequence as an N-mer within the product composition (either in a single nucleic acid member of the product or in a "concatamer" of two different nucleic acid members of the product), then the product composition is considered to be a composition of non-reduced complexity as compared to the initial source.

In many embodiments, the length N of the sequence (i.e., N-mer) that is randomly chosen from the initial source ranges from about 45 to about 200 nt, including from about 50 to about 100 nt, such as from about 55 to about 65 nt, e.g., 60 nt. For example, if a sequence of 60 nt in length that is randomly chosen uniformly over an initial genomic source sequence has a high probability of being in the product composition, then the product composition has a non-reduced complexity as compared to the parent composition. For this purpose, a given sequence is considered to have a high probability of being in a product composition if its probability of being in the product composition, either in a single nucleic acid member or in a concatamer of two different members, is at least about 10%, for example at least about 25%, including at least about 50%, where in certain embodiments the probability may be about 60%, about 70%, about 80%, about 90%, about 95% or higher, e.g., about 98%, etc. With knowledge of the sequence within the genomic source and product, the probability that a given sequence randomly chosen from the initial source is present in a given product composition may be determined according to the following parameters:

Consider a nucleotide sequence of the genomic source: G. Consider a fixed integer N. Consider a collection of nucleic acids, M = {$m_1$, $m_2$, ... , $m_k$} where each $m_i$ is a subsequence of G. For any N-mer sequence w, define

$$\sigma_G(w) = \begin{cases} 1 & w \text{ is a subsequence of } G \\ 0 & \text{otherwise} \end{cases}$$

$$\sigma_M(w) = \begin{cases} 1 & \begin{array}{c} w \text{ is a subsequence of some } m_i \text{ or} \\ \text{of some concatenation } m_i * m_j \end{array} \\ 0 & \text{otherwise} \end{cases}$$

Set

$$S_G = \sum_{N-mers} \sigma_G(w)$$

and

$$S_M = \sum_{N-mers} \sigma_M(w)$$

Where the sums are over all mathematically possible N-mers. The probability that a random N-mer W uniformly selected over G is present in M is then

$$p = \frac{S_M}{S_G}.$$

From a practical point of view, the numbers SM and SG can be computed by stepping along the sequences and incrementing by 1 every time a new N-mer is visited. Then all pairs of concatemers from M are also processed in the same way. Given the formulas, this calculation is then obvious to anyone skilled in the art of programming.

A non-reduced complexity collection of nucleic acids can be readily identified using a number of different protocols. One convenient protocol for determining whether a given collection of nucleic acids is a non-reduced complexity collection of nucleic acids is to screen the collection using a genome wide array of features for the initial, e.g., genomic source of interest. Thus, one can tell whether a given collection of nucleic acids has non-reduced complexity with respect to its genomic source by assaying the collection with a genome wide array for the genomic source. The genome wide array of the genomic source for this purpose is an array of features in which the collection of features of the array used to test the sample is made up of sequences uniformly and independently randomly chosen from the initial genomic source. As such, sequences of sufficient length, e.g., N length as described above, independently chosen randomly from the initial nucleic acid source that uniformly sample the initial nucleic acid source are present in the collection of features on the array. By uniformly is meant that no bias is present in the selection of sequences from the initial genomic source. In such a genome wide assay of sample, a non-reduced complexity sample is one in which substantially all of the array features on the array specifically hybridize to nucleic acids present in the sample, where by substantially all is meant at least about 10%, for example at least about 25%, including at least about 50%, such as at least about 60, 70, 75, 80, 85, 90 or 95% or more.

As such, according to the above guidelines, a sample is considered to be of non-reduced complexity as compared to its genomic source if its complexity is at least about 10%, for example at least about 25%, including at least about 50%, such as at least about 60, 70, 75, 80, 85, 90 or 95% or more of the complexity of the genomic source, as detailed above.

In certain other embodiments, however, a population of labeled nucleic acids may be one that is of reduced complexity as compared to the initial genomic extract. By reduced complexity is meant that the complexity of the produced population of nucleic acids is at least about 20-fold less, such as at least about 25-fold less, at least about 50 -fold less, at least about 75-fold less, at least about 90-fold less, at least about 95-fold less complex, than the complexity of the initial genomic extract, in terms of total numbers of sequences found in the produced population of labeled nucleic acids as compared to the initial genomic extract, up to and including a single CpG island being represented in the population. Examples of protocols that can produce reduced complexity product compositions of utility in genotyping and gene expression include those described in U.S. Patent No. 6,465,182 and published PCT application WO 99/23256; as well as published U.S. Patent Application No. 2003/0036069 and Jordan et al., Proc. Nat'l Acad. Sci. USA (March 5, 2002) 99: 2942-2947. In each of these protocols that produce a reduced complexity product, primers are employed that have been designed to knowingly produce product nucleic acids from only a select fraction or portion of the initial genomic source, e.g., genome, where fraction or portion may be defined as a subset or representative subset of a genome.

Accordingly, in many embodiments, at least a first population of labeled nucleic acids and a second population of labeled nucleic acids are produced from two different genomic samples, e.g., one digested with a methylation-sensitive restriction enzyme and the other not digested with such an enzyme. As indicated above, depending on the particular assay protocol (e.g., whether both populations are to be hybridized simultaneously to a single array or whether each population is to be hybridized to two different but substantially identical, if not identical, arrays) the populations may be labeled with the same or different labels. As such, a feature of certain embodiments is that the different populations of labeled probe nucleic acids are labeled with the same label, such that they are not distinguishably labeled. In yet other embodiments, a feature of the different populations of labeled nucleic acids is that the first and second labels are typically distinguishable from each other. The constituent probe members of the above produced collections typically range in length from about 100 to about 1000 nt, such as from about 200 to about 800 nt, including from about 300 to 500 nt, etc.

The labeling reactions produce a first and second population of labeled nucleic acids that correspond to the digested and undigested target compositions, respectively. After nucleic acid purification and any pre-hybridization steps to suppress repetitive sequences (e.g., hybridization with Cot-1 DNA), the populations of labeled nucleic acids are usually contacted to an array of surface-bound oligonucleotides, as discussed above, under conditions such that nucleic acid hybridization to the surface-bound oligonucleotides can occur, e.g., in a buffer containing 50% formamide, 5×SSC and 1% SDS at 42°C, or in a buffer containing 5×SSC and 1% SDS at 65°C, both with a wash of 0.2×SSC and 0.1 % SDS at 65°C.

The collections can be contacted to the surface immobilized elements either simultaneously or serially. In many embodiments the nucleic acids are contacted with a subject array simultaneously. Depending on how the populations are labeled, the populations may be contacted with the same array or different arrays, where when the populations are contacted with different arrays, the different arrays are substantially, if not completely, identical to each other in terms of feature content and organization.

Standard hybridization techniques (using high stringency hybridization conditions) are used to probe subject array. Suitable methods are described in many references (e.g., Kallioniemi et al., Science 258:818-821 (1992) and WO 93/18186). Several guides to general techniques are available, e.g., Tijssen, Hybridization with Nucleic Acid Probes, Parts I and II (Elsevier, Amsterdam 1993). For a descriptions of techniques suitable for *in situ* hybridizations see, Gall et al. Meth. Enzymol., 21:470-480 (1981) and Angerer et al. in Genetic Engineering: Principles and Methods Setlow and Hollaender, Eds. Vol 7, pgs 43-65 (plenum Press, New York 1985). See also United States Patent Nos: 6,335,167; 6,197,501; 5,830,645; and 5,665,549; the disclosures of which are herein incorporate by reference.

Generally, the subject methods comprise the following major steps: (1) provision of an array of subject surface-bound CpG UNA oligonucleotides; (2) pre-hybridization treatment to increase accessibility of surface-bound CpG UNA oligonucleotides, and to reduce nonspecific binding; (3) hybridization of a population of labeled nucleic acids to the surface-bound CpG UNA oligonucleotides, typically under high stringency conditions; (4) post-hybridization washes to remove nucleic acids not bound in the hybridization; and (5) detection of the hybridized nucleic acids. The reagents used in each of these steps and their conditions for use vary depending on the particular application.

Optionally, prior to step (3), the complexity of the population of labeled nucleic acids may be reduced by a pre-incubation step, e.g., hybridized with nucleic acids to suppress repetitive or unwanted sequences. In some embodiments, Cot-1 nucleic acids may be used. However, in certain embodiments were it is desirable to suppress certain repetitive sequences but not others, the population of labeled nucleic acids may by pre-incubated with certain types of nucleic acids for suppressing only those undesirable sequences. For example, the population of labeled nucleic acids may be incubated with a mixture of nucleic acids containing any repetitive sequences, e.g., Alu, LINE (e.g., LINE-1), SINE (e.g., SINE B1 and B2), or microsatellite repeat sequences.

As indicated above, hybridization is carried out under suitable hybridization conditions, which may vary in stringency as desired. In certain embodiments, highly stringent hybridization conditions may be employed. The term "highly stringent hybridization conditions" as used herein refers to conditions that are compatible to produce nucleic acid binding complexes on an array surface between complementary binding members, i.e., between surface-bound subject oligonucleotides and complementary labeled nucleic acids in a sample. Representative high stringency assay conditions that may be employed in these embodiments are provided above. In most embodiments, a subject CpG UNA oligonucleotide will hybridize to an intact, uncleaved target CpG island, but not a cleaved target CpG island under highly stringent conditions.

The above hybridization step may include agitation of the immobilized targets and the sample of labeled nucleic acids, where the agitation may be accomplished using any convenient protocol, e.g., shaking, rotating, spinning, and the like.

Following hybridization, the surface of immobilized nucleic acids is typically washed to remove unbound labeled nucleic acids. Washing may be performed using any convenient washing protocol, where the washing conditions are typically stringent, as described above.

Following hybridization and washing, as described above, the hybridization of the labeled nucleic acids to the array is then detected using standard techniques so that the surface of the array, is read. Reading of the resultant hybridized array may be accomplished by illuminating the array and reading the location and intensity of resulting fluorescence at each feature of the array to detect any binding complexes on the surface of the array. For example, a scanner may be used for this purpose that is similar to the AGILENT MICROARRAY SCANNER available from Agilent Technologies, Palo Alto, CA. Other suitable devices and methods are described in U.S. patent applications: Serial No. 09/846125 "Reading Multi-Featured Arrays" by Dorsel et al.; and United States Patent No. 6,406,849, which references are incorporated herein by reference. However, arrays may be read by any other method or apparatus than the foregoing, with other reading methods including other optical techniques (for example, detecting chemiluminescent or electroluminescent labels), or electrical techniques (where each feature is provided with an electrode to detect hybridization at that feature in a manner disclosed in US 6,221,583 and elsewhere). In the case of indirect labeling, subsequent treatment of the array with the appropriate reagents may be employed to enable reading of the array. Some methods of detection, such as surface plasmon resonance, do not require any labeling of the probe nucleic acids, and are suitable for some embodiments.

Results from the reading or evaluating may be raw results (such as fluorescence intensity readings for each feature in one or more color channels) or may be processed results (such as those obtained by subtracting a background measurement, or by rejecting a reading for a feature which is below a predetermined threshold, normalizing the results, and/or forming conclusions based on the pattern read from the array (such as whether or not a particular target sequence may have been present in the sample, or whether or not a pattern indicates a particular condition of an organism from which the sample came).

In certain embodiments, the subject methods include a step of transmitting data or results from at least one of the detecting and deriving steps, also referred to herein as evaluating, as described above, to a remote location. By "remote location" is meant a location other than the location at which the array is present and hybridization occur. For example, a remote location could be another location (e.g. office, lab, etc.) in the same city, another location in a different city, another location in a different state, another location in a different country, etc. As such, when one item is indicated as being "remote" from another, what is meant is that the two items are at least in different buildings, and may be at least one mile, ten miles, or at least one hundred miles apart.

"Communicating" information means transmitting the data representing that information as electrical signals over a suitable communication channel (for example, a private or public network). "Forwarding" an item refers to any means of getting that item from one location to the next, whether by physically transporting that item or otherwise (where that is possible) and includes, at least in the case of data, physically transporting a medium carrying the data or communicating the data. The data may be transmitted to the remote location for further evaluation and/or use. Any convenient telecommunications means may be employed for transmitting the data, e.g., facsimile, modem, internet, etc.

In certain embodiments, CpG island methylation is assessed by determining a level of binding of the population of labeled nucleic acids to a subject oligonucleotide feature corresponding to that CpG island. The term "level of binding" means any assessment of binding (e.g. a quantitative or qualitative, relative or absolute assessment) usually done, as is known in the art, by detecting signal (i.e., pixel brightness) from the label associated with the labeled nucleic acids. Since the level of binding of labeled nucleic acid to a subject oligonucleotide feature is proportional to the level of bound label, the level of binding of labeled nucleic acid is usually determined by assessing the amount of label associated with the feature.

In certain embodiments, a CpG island methylation may be assessed by evaluating winding of a subject oligonucleotide feature corresponding to that CpG island to two populations of nucleic acids that are distinguishably labeled. In these embodiments, for a single subject oligonucleotide feature, the results obtained from hybridization with a first population of labeled nucleic acids may be compared to results obtained from hybridization with the second population of nucleic acids, usually after normalization of the data. The results may be expressed using any convenient means, e.g., as a number or numerical ratio, etc.

By "normalization" is meant that data corresponding to the two populations of nucleic acids are globally normalized to each other, and/or normalized to data obtained from controls (e.g., internal controls produce data that are predicted to equal in value in all of the data groups). Normalization generally involves multiplying each numerical value for one data group by a value that allows the direct comparison of those amounts to amounts in a second data group. Several normalization strategies have been described (Quackenbush et al, Nat Genet. 32 Suppl:496-501, 2002, Bilban et al Curr Issues Mol Biol. 4:57-64, 2002, Finkelstein et al, Plant Mol Biol.48(1-2):119-31, 2002, and Hegde et al, Biotechniques. 29:548-554, 2000). Specific examples of normalization suitable for use in the subject methods include linear normalization methods, non-linear normalization methods, e.g., using lowess local regression to paired data as a function of signal intensity, signal-dependent non-linear normalization, qspline normalization and spatial normalization, as described in Workman et al., (Genome Biol. 2002 3, 1-16). In certain embodiments, the numerical value associated with a feature signal is converted into a log number, either before or after normalization occurs. Data may be normalized to data obtained using the data obtained from a support-bound polynucleotide for a CpG island of known methylation in the target compositions.

Accordingly, CpG island methylation may be assessed by detecting binding of a subject oligonucleotide feature to a labeled population of nucleic acids. In most embodiments, the assessment provides a numerical assessment of binding, and that numeral may correspond to an absolute level of binding, a relative level of binding, or a qualitative (e.g., presence or absence) or a quantitative level of binding. Accordingly, a binding assessment may be expressed as a ratio, whole number, or any fraction thereof.

In other words, any binding may be expressed as the level of binding of a subject oligonucleotide feature to a labeled population of nucleic acids made from a target composition, divided by its level of binding to a labeled population of nucleic acids made from a control for the test sample (or vice versa). This number provides an accurate estimate of methylation of a CpG island in a cell. In one protocol the control consists of an aliquot of the target composition that is not contacted with the methylation sensitive restriction enzyme. In this example, if a ratio approaches zero for a particular subject oligonucleotide feature, the CpG island corresponding to that oligonucleotide is likely to be unmethylated. Similarly, any obtained ratio significantly above zero indicates that the CpG island is methylated. An increase in this ratio indicates a proportional increase in the extent of methylation of a particular CpG island for a sample of interest.

Particular embodiments of the invention are set forth schematically in Fig. 2. A sample containing a methylated CpG island (top) is digested with a methylation-sensitive restriction enzyme, labeled, and hybridized to a subject array. Binding of the labeled sample to a subject oligonucleotide in the array is assessed, and any binding of the labeled sample indicates the presence of a methylated CpG island. Suitable controls are shown in the remainder of Fig. 2, and include (in the middle and right), controls in which the sample is not digested with a methylation-sensitive enzyme (middle), labeled, and hybridized to a subject array. Since this sample is undigested, this control should provide a total number of CpG islands in a sample, methylated or not. The results obtained from a test sample (on the left), indicating a level of a methylated CpG island may be compared to these results (indicating the total amount of that CpG island, methylated or not), and a resultant fraction indicating the fraction of total CpG islands that are methylated, may be obtained. A second control (shown on the right) involves digesting the sample with a methylation-insensitive restriction enzyme. In this control, no significant binding to the subject oligonucleotide should occur since all of the CpG islands for that oligonucleotide are cleaved.

Accordingly, since, the arrays used in the subject assays may contain a subject oligonucleotides for a plurality of different CpG islands, methylation of those CpG islands may be assessed. The subject methods are therefore suitable for simultaneous assessment of the methylation of a large number of CpG islands.

In alternative embodiments, after contacting the CpG island composition with a methylation-sensitive restriction enzyme and a restriction enzyme that cleaves outside of CpG islands, linkers may be added, and the restriction products may be amplified and labeled using so-called "differential methylation hybridization" (DMH) methods, 20030129602 and Huang et al., (Human Mol. Genet. (1999) 8: 459-70), and hybridized to the subject arrays to assess methylation of a CpG island. The use of arrays containing UNA oligonucleotides improves the sensitivity of DMH methods.

## METHODS OF COMPARING CpG ISLAND METHYLATION STATUS

The invention provides methods of comparing methylation of a CpG island in a reference cell and a test cell. In general, the methods involve employing the methods set forth above to evaluate CpG island methylation in the reference and test cells. In most embodiments, the methods involve independently contacting genomic samples from a reference cell and a test cell with a methylation-sensitive restriction enzyme to make reference and test target compositions, and assessing binding of the reference and test compositions to a subject CpG UNA oligonucleotide. In certain embodiments, the reference and test compositions may be contacted to the same or different array and compared directly. In other embodiments, methylation of a CpG island in the reference and test compositions are first assessed relative to suitable controls, as described in the previous section.

For example, in certain embodiments and with reference to Fig. 3, genomic samples may be prepared from the reference and test cells, the samples contacted with a suitable methylation-sensitive restriction enzyme to make target compositions, and the target compositions distinguishably labeled and hybridized to a subject oligonucleotide. In these embodiments, the relative binding of the labeled target compositions to the oligonucleotide indicates the relative level of methylation of a CpG island in those cells. For example, if a ratio of about 1 is obtained, the CpG island is methylated at similar levels in both of the cells. If a ratio of less than or greater than 1 is obtained, the CpG island is methylated to a greater extent in one of the cells compared to the other.

In other embodiments, genomic samples made from the reference and test cells may be independently assessed relative to suitable controls, e.g., genomic sample from the cells that have not been contacted with a methylation-sensitive restriction enzyme, as discussed above, to provide an assessment of methylation of a CpG island for both of the cells. For example, one cell may contain a CpG island that is 10% methylated, whereas the other cell may contain a CpG island that is 90% methylated. By comparing these figures, the level of methylation of a CpG island can be compared between two different cells.

Accordingly, the subject methods may be used to detect changes in methylation status in cells, and abnormal methylation, i.e., "hypomethylation" or "hypermethylation", which terms are well known and used in the art.

The test and reference cell of a test and reference cell pair may be any two cells. However, in many embodiments, one cell of the pair has or is suspected of having a different phenotype compared to the other cell. In a particular embodiment, test and reference cell pairs include cancerous cells, e.g., cells that exhibit increased proliferation, and non-cancerous cells, respectively or cells obtained from a sample of tissue from a test subject, e.g., a subject suspected of having a CpG island methylation abnormality, and cells obtained from a normal, reference subject, respectively.

Accordingly, cells from yeast, plants and animals, such as fish, birds, reptiles, amphibians and mammals may be used in the subject methods. In certain embodiments, mammalian cells, i.e., cells from mice, rabbits, primates, or humans, or cultured derivatives thereof, may be used.

## COMPUTER-RELATED EMBODIMENTS

The invention also provides a variety of computer-related embodiments. Specifically, the methods of analyzing data to assess CpG island methylation described in the previous section may be performed using a computer. Accordingly, the invention provides a computer-based system for assessing CpG island methylation using the above methods.

In most embodiments, the methods are coded onto a computer-readable medium in the form of "programming", where the term "computer readable medium" as used herein refers to any storage or transmission medium that participates in providing instructions and/or data to a computer for execution and/or processing. Examples of storage media include floppy disks, magnetic tape, CD-ROM, a hard disk drive, a ROM or integrated circuit, a magneto-optical disk, or a computer readable card such as a PCMCIA card and the like, whether or not such devices are internal or external to the computer. A file containing information may be "stored" on computer readable medium, where "storing" means recording information such that it is accessible and retrievable at a later date by a computer.

With respect to computer readable media, "permanent memory" refers to memory that is permanent. Permanent memory is not erased by termination of the electrical supply to a computer or processor. Computer hard-drive ROM (i.e. ROM not used as virtual memory), CD-ROM, floppy disk and DVD are all examples of permanent memory. Random Access Memory (RAM) is an example of non-permanent memory. A file in permanent memory may be editable and re-writable.

A "computer-based system" refers to the hardware means, software means, and data storage means used to analyze the information of the present invention. The minimum hardware of the computer-based systems of the present invention comprises a central processing unit (CPU), input means, output means, and data storage means. A skilled artisan can readily appreciate that any one of the currently available computer-based system are suitable for use in the present invention. The data storage means may comprise any manufacture comprising a recording of the present information as described above, or a memory access means that can access such a manufacture.

To "record" data, programming or other information on a computer readable medium refers to a process for storing information, using any such methods as known in the art. Any convenient data storage structure may be chosen, based on the means used to access the stored information. A variety of data processor programs and formats can be used for storage, e.g. word processing text file, database format, etc.

A "processor" references any hardware and/or software combination which will perform the functions required of it. For example, any processor herein may be a programmable digital microprocessor such as available in the form of a electronic controller, mainframe, server or personal computer (desktop or portable). Where the processor is programmable, suitable programming can be communicated from a remote location to the processor, or previously saved in a computer program product (such as a portable or fixed computer readable storage medium, whether magnetic, optical or solid state device based). For example, a magnetic medium or optical disk may carry the programming, and can be read by a suitable reader communicating with each processor at its corresponding station.

## KITS

Also provided by the subject invention are kits for practicing the subject methods, as described above. The subject kits at least include a CpG UNA oligonucleotide that may be surface-bound to a planar solid support. Other optional components of the kit include: a methylation-sensitive enzyme, a methylation-insensitive isoschizomer of that enzyme, an enzyme that has a cleavage site generally outside of CpG islands, nucleic acid labeling agents, such as primer extension or nick translation and fluorescent labels conjugated to nucleotides, Cot-1 or other suppressors or repetitive DNA, and control or reference compositions for use in testing the other compositions of the kit. In some embodiments, arrays may be included in the kits. In alternative embodiments, the kit may also contain computer-readable media for performing the subject methods, as discussed above. The various components of the kit may be present in separate containers or certain compatible components may be precombined into a single container, as desired.

In addition to above-mentioned components, the subject kits typically further include instructions for using the components of the kit to practice the subject methods. The instructions for practicing the subject methods are generally recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or subpackaging) etc. In other embodiments, the instructions are present as an electronic storage data file present on a suitable computer readable storage medium, e.g. CD-ROM, diskette, etc. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, this means for obtaining the instructions is recorded on a suitable substrate.

In addition to the subject database, programming and instructions, the kits may also include one or more control analyte mixtures, e.g., two or more control compositions for use in testing the kit.

## UTILITY

The above-described compositions and methods find use in any application in which one wishes to assess CpG island methylation in a cell. One type of representative application in which the subject methods find use is the quantitative comparison of level of CpG island methylation in a first cell relative to the level of the same CpG island in a second cell, i.e., detecting the relative methylation levels of a CpG island a cell (see, e.g., Fig. 3). Since the subject methods may be performed using a plurality of subject oligonucleotides in an array, the subject methods find most use in assessing global changes in methylation patterns between two cell types.

The subject invention therefore finds use in methods for detecting differences in CpG methylation between two cells and, accordingly, finds particular use as a diagnostic and research tool for investigating diseases, conditions and other subjects of interest relating to CpG methylation, e.g., cancer, embryonic development, X-inactivation, genomic imprinting, regulation of gene expression, and host defense against parasitic sequences, fragile site expression, and cytosine to thymine transition mutations. In particular embodiments, once abnormally methylated CpG islands are identified, the expression of genes proximal to the CpG islands may be investigated.

In general, two populations of labeled nucleic acids, representing a test and reference cells, are hybridized with a subject array as discussed above. The arrays are washed and read to provide data, and that data provides information on the relative methylation of at least one CpG island in the test and reference cells. In some embodiments, assuming that the reference cell is "normal", any results that indicate that a particular methylated CpG island is present at a greater amount in a test cell, relative to that of the reference cell, indicates that the CpG island has abnormally methylated, i.e., hypermethylated, in the test cell. Conversely, any results that indicate that a particular methylated CpG island is present at a lower amount in a test cell, relative to that of the reference cell, indicates that the CpG island is hypomethylated in the test cell.

The following examples are offered by way of illustration and not by way of limitation.

## EXPERIMENTAL

### *MATERIALS AND METHODS*

**Sample preparation.** Genomic DNA is prepared from a tumor sample using the DNeasy Tissue Kit (Qiagen, Germantown, MD). For each CGH hybridization, 40 μg of genomic DNA is digested with *Alu*I (12.5 units) and *Rsa*I (12.5 units) (Promega). One half (20 ug) of each sample is then digested with *Hpa*II (Promega) All digests are done for a minimum of 2 hours at 37°C and verified by agarose gel analysis. Samples are then filtered using the Qiaquick PCR Cleanup Kit (Qiagen). Labeling reactions are performed with 6 μg of purified restricted DNA and a Bioprime labeling kit (Invitrogen) according to the manufacturer's directions in a 50 μl volume with a modified dNTP pool; 120 μM each of dATP, dGTP, dTTP, 60 μM dTTP, and 60 μM of either Cy5-dUTP for the *Hpa*II digested sample or Cy3-dUTP for the reference sample that is not treated with *Hpa*II. Labeled targets are subsequently filtered using a Centricon YM-30 filter (Millipore, Bedford, MA). Targets for each hybridization are pooled, mixed with competitor DNA (Invitrogen), 100 μg of yeast tRNA (Invitrogen) and 1X hybridization control targets (SP310, Operon). The target mixture is purified then concentrated with a Centricon YM-30 column, and resuspended to a final volume of 250 μl, then mixed with an equal volume of Agilent 2X *in situ* Hybridization Buffer.

### *RESULTS*

Exemplary hypothetical results showing the methylation status of a CpG island adjacent to the human Asparagine Synthetase (*AS*) gene is shown in Figure 4. The intact target sequence binds to the probe under high stringency hybridization and wash conditions (Figure 4a).CGH analysis of a tumor sample with UNA oligonucleotides for *AS* detects a ratio value close to 1.0 for the methylated CpG island relative to the intact non *Hpa*II digested control sample (Figure 4a). In contrast, the digested target sequences do not bind efficiently under the same hybridization and wash conditions. The CGH analysis of a normal cell sample detects a ratio value of 0.1 for the same CpG island (Figure 4b). Thus these normal cells are unmethylated while the tumor cells have methylated copies of the *AS* CpG island.

The above results and discussion demonstrate a new method for assessing methylation of CpG islands in a cell. Such methods are superior to currently used methods because they provide a high-throughput genome-wide way of directly and accurately quantifying the methylation status of CpG islands in a cell using a CpG UNA oligonucleotide. The CpG UNA oligonucleotide, because it has reduced secondary structure, provides better, more reliable results than conventional oligonucleotides. Because the subject methods rely on CpG UNA oligonucleotides, secondary structure effects can be minimized while maintaining maximum hybridization affinity, and several CpG UNA oligonucleotides may be straightforwardly designed and used to assay the methylation state of several, if not all, CpG islands in parallel. As such, the subject methods represent a significant contribution to the art.

All publications and patent applications cited in this specification are herein incorporated by reference as if each individual publication or patent application were specifically and individually indicated to be incorporated by reference. The citation of any publication is for its disclosure prior to the filing date and should not be construed as an admission that the present invention is not entitled to antedate such publication by virtue of prior invention.

Although the foregoing invention has been described in some detail by way of illustration and example for purposes of clarity of understanding, it is readily apparent to those of ordinary skill in the art in light of the teachings of this invention that certain changes and modifications may be made thereto without departing from the scope of the appended claims.

The disclosures in United States patent application No. 10/828,986, from which this application claims priority, and in the abstract accompanying this application are incorporated herein by reference.

## Claims

1. A CpG unstructured nucleic acid (UNA) oligonucleotide.

2. An oligonucleotide as claimed in claim 1, wherein said CpG UNA oligonucleotide binds to an uncleaved CpG island, but not to a CpG island cleaved by a methylation-sensitive restriction enzyme, under stringent hybridization conditions.

3. A oligonucleotide as claimed in claim 1 or 2, wherein said oligonucleotide comprises nucleotides G' and C', wherein said nucleotides G' and C' base pair with each other with a stability that is lower than that of G and C.

4. An array of features comprising at least one feature comprising an oligonucleotide as claimed in claim 1, 2 or 3.

5. A method for evaluating methylation of a CpG island, comprising
   contacting said CpG island with a methylation-sensitive restriction enzyme to produce a target composition; and
   assessing binding of said target composition to a CpG UNA oligonucleotide as claimed in any of claims 1 to 3.

6. A method of comparing methylation of a CpG island in a reference cell and a test cell, comprising:

   employing the method of claim 5 to evaluate methylation of said CpG island in said reference and test second cells independently; and
   comparing the results of said evaluation.

7. A method of assaying methylation of CpG islands in a sample comprising:

(a) contacting a sample with a methylation sensitive restriction enzyme;
(b) contacting an array as claimed in claim 4 with the composition produced by step (a); and
(c) detecting the presence of any resultant binding complexes on the surface of said array.

**8.** A method comprising transmitting data from a method as claimed in claim 7 from a first location to a remote location.

**9.** A method comprising receiving a transmitted result of a reading of an array obtained according to a method as claimed in claim 7.

**10.** A kit comprising:

a CpG island unstructured nucleic acid (UNA) oligonucleotide.

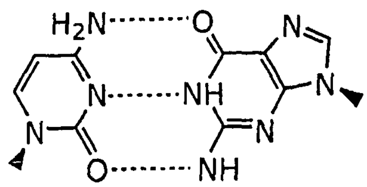

C - G

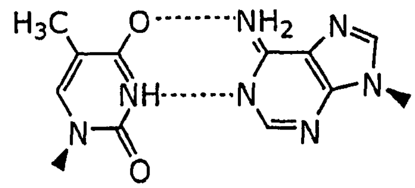

T (or U) - A

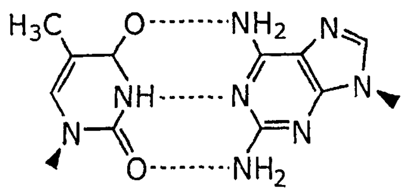

T (or U) - 2-amino A

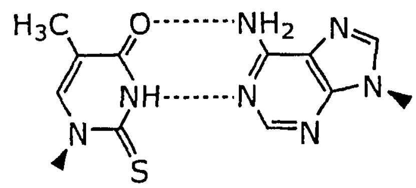

2-thio T (or U) - A

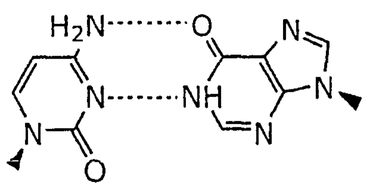

C - I

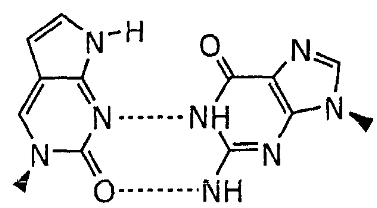

PyrroloPyr - G

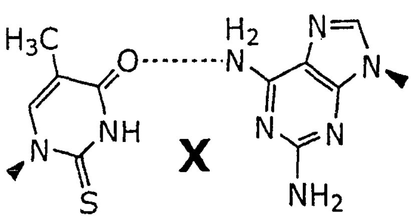

2-thio T (or U) - 2-amino A

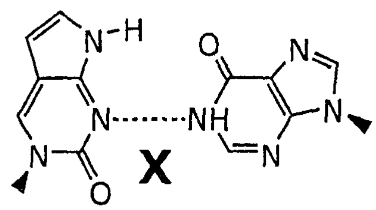

PyrroloPyr - I

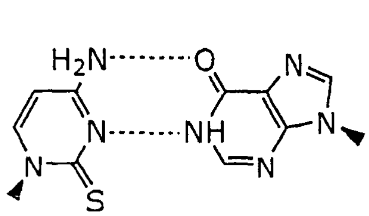

2-thio C - I

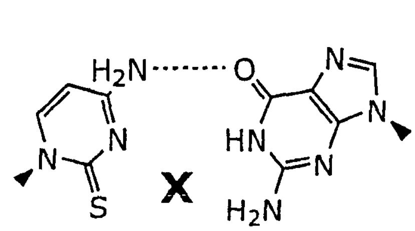

2-thio C - G

FIG. 1

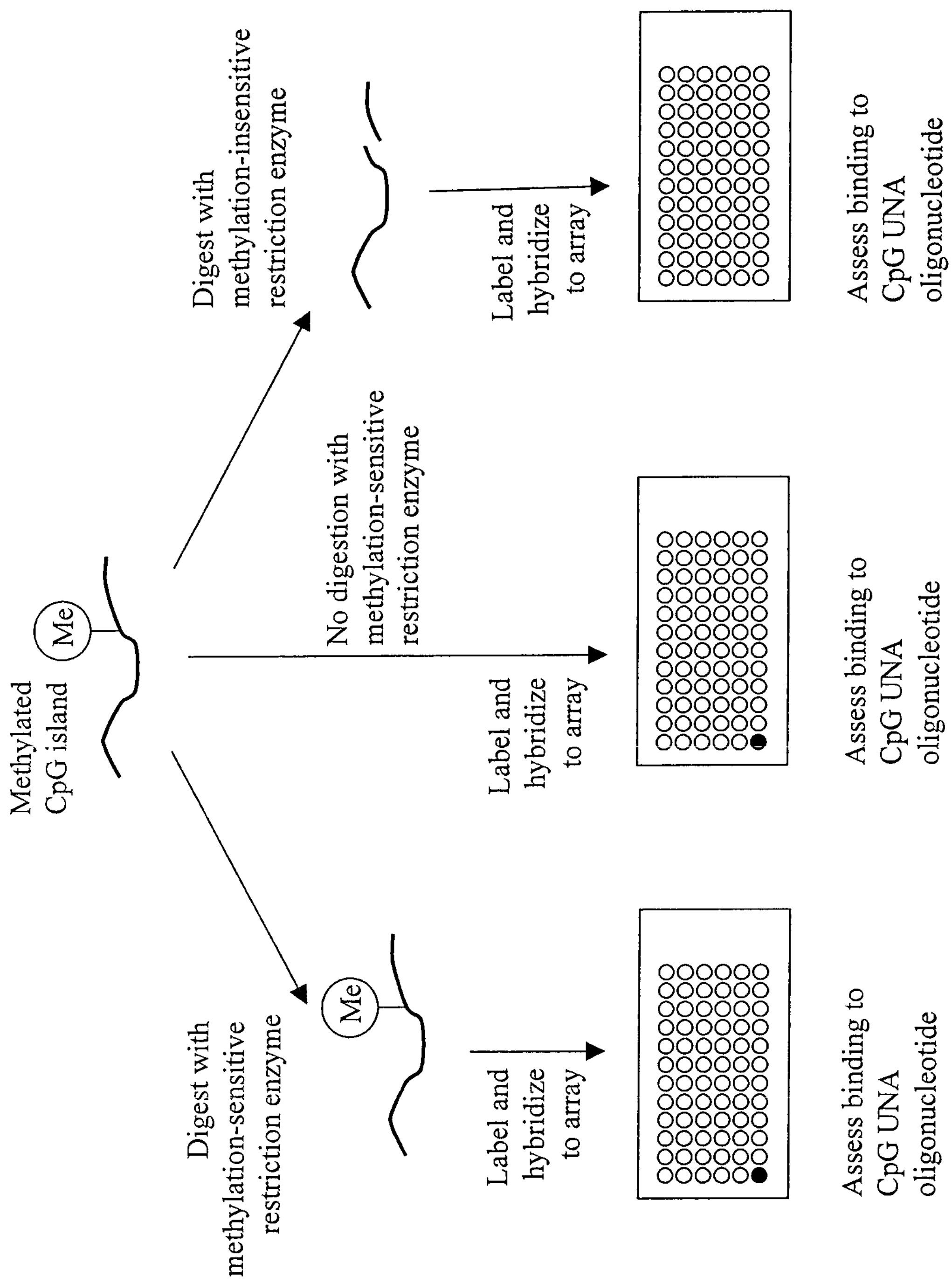
Methylated CpG island
Me
Digest with methylation-sensitive restriction enzyme
Me
Label and hybridize to array
Assess binding to CpG UNA oligonucleotide
No digestion with methylation-sensitive restriction enzyme
Label and hybridize to array
Assess binding to CpG UNA oligonucleotide
Digest with methylation-insensitive restriction enzyme
Label and hybridize to array
Assess binding to CpG UNA oligonucleotide

FIG. 2

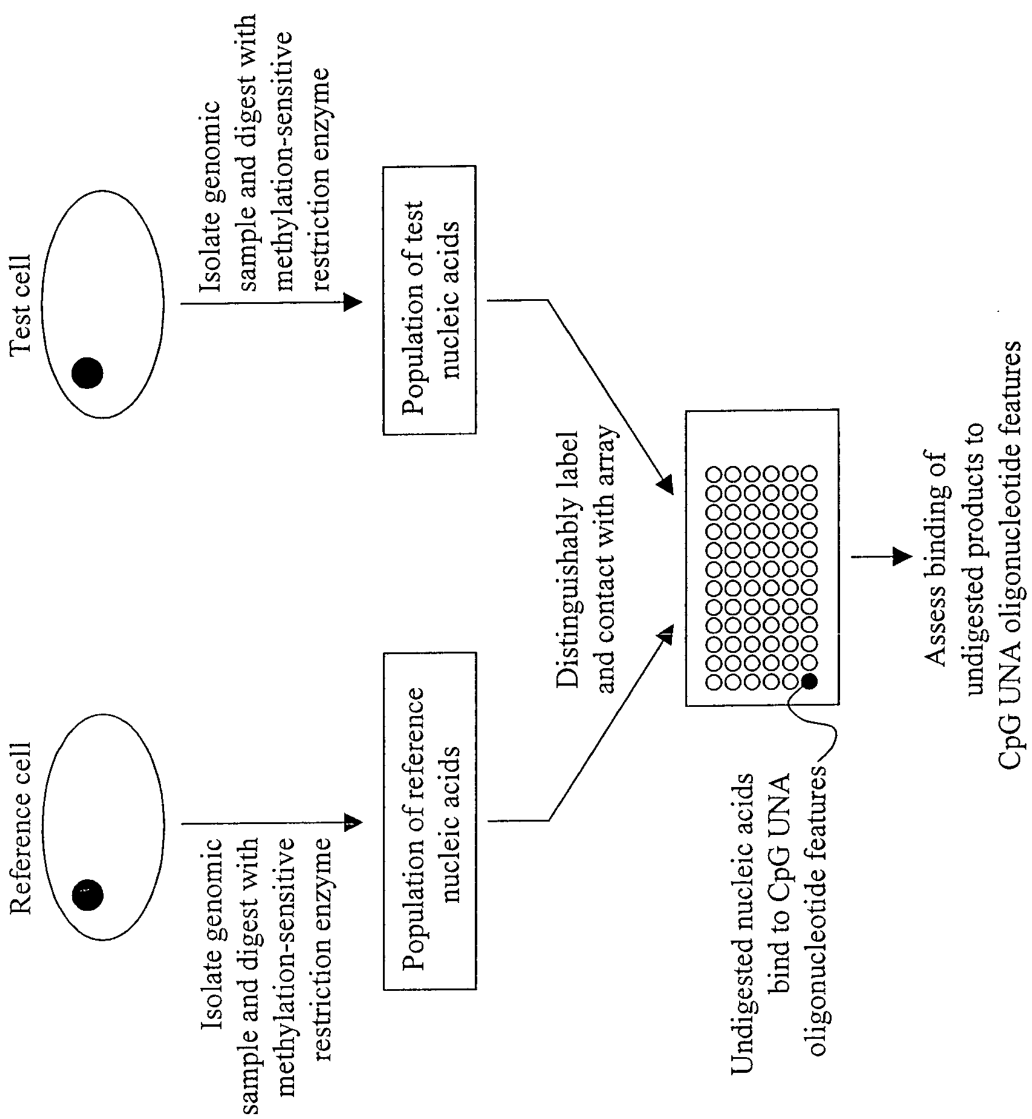
Reference cell
Test cell
Isolate genomic sample and digest with methylation-sensitive restriction enzyme
Isolate genomic sample and digest with methylation-sensitive restriction enzyme
Population of reference nucleic acids
Population of test nucleic acids
Distinguishably label and contact with array
Undigested nucleic acids bind to CpG UNA oligonucleotide features
Assess binding of undigested products to CpG UNA oligonucleotide features

FIG. 3

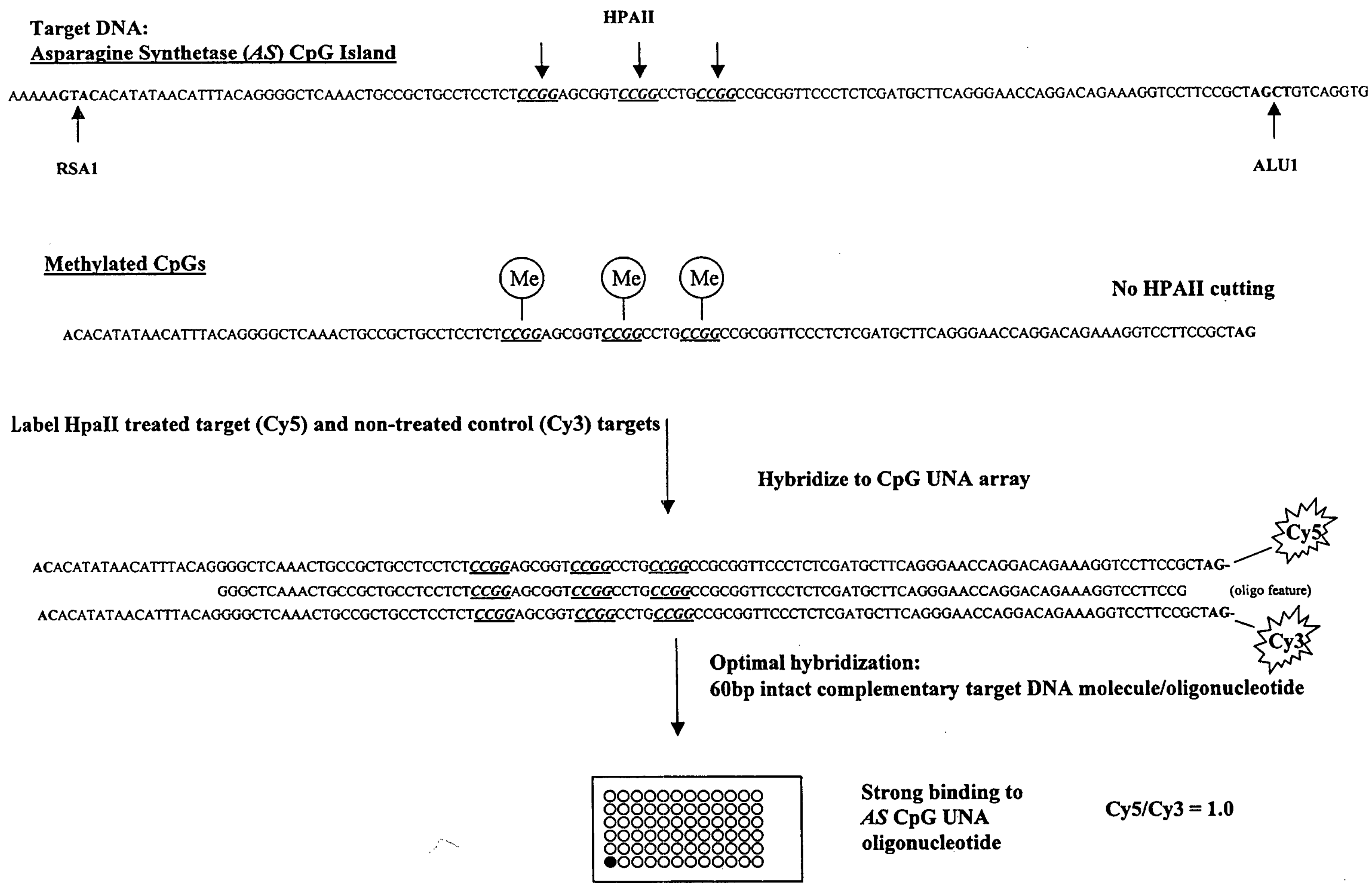
Target DNA:
Asparagine Synthetase (AS) CpG Island
HPAII
AAAAAGTACACATATAACATTTACAGGGGCTCAAACTGCCGCTGCCTCCTCTCCGGAGCGGTCCGGCCTGCCGGCCGCGGTTCCCTCTCGATGCTTCAGGGAACCAGGACAGAAAGGTCCTTCCGCTAGCTGTCAGGTG
RSA1
ALU1
Methylated CpGs
Me
Me
Me
No HPAII cutting
ACACATATAACATTTACAGGGGCTCAAACTGCCGCTGCCTCCTCTCCGGAGCGGTCCGGCCTGCCGGCCGCGGTTCCCTCTCGATGCTTCAGGGAACCAGGACAGAAAGGTCCTTCCGCTAG
Label HpaII treated target (Cy5) and non-treated control (Cy3) targets
Hybridize to CpG UNA array
ACACATATAACATTTACAGGGGCTCAAACTGCCGCTGCCTCCTCTCCGGAGCGGTCCGGCCTGCCGGCCGCGGTTCCCTCTCGATGCTTCAGGGAACCAGGACAGAAAGGTCCTTCCGCTAG-
Cy5
GGGCTCAAACTGCCGCTGCCTCCTCTCCGGAGCGGTCCGGCCTGCCGGCCGCGGTTCCCTCTCGATGCTTCAGGGAACCAGGACAGAAAGGTCCTTCCG
(oligo feature)
ACACATATAACATTTACAGGGGCTCAAACTGCCGCTGCCTCCTCTCCGGAGCGGTCCGGCCTGCCGGCCGCGGTTCCCTCTCGATGCTTCAGGGAACCAGGACAGAAAGGTCCTTCCGCTAG-
Cy3
Optimal hybridization:
60bp intact complementary target DNA molecule/oligonucleotide
Strong binding to
AS CpG UNA
oligonucleotide
Cy5/Cy3 = 1.0

FIG. 4A

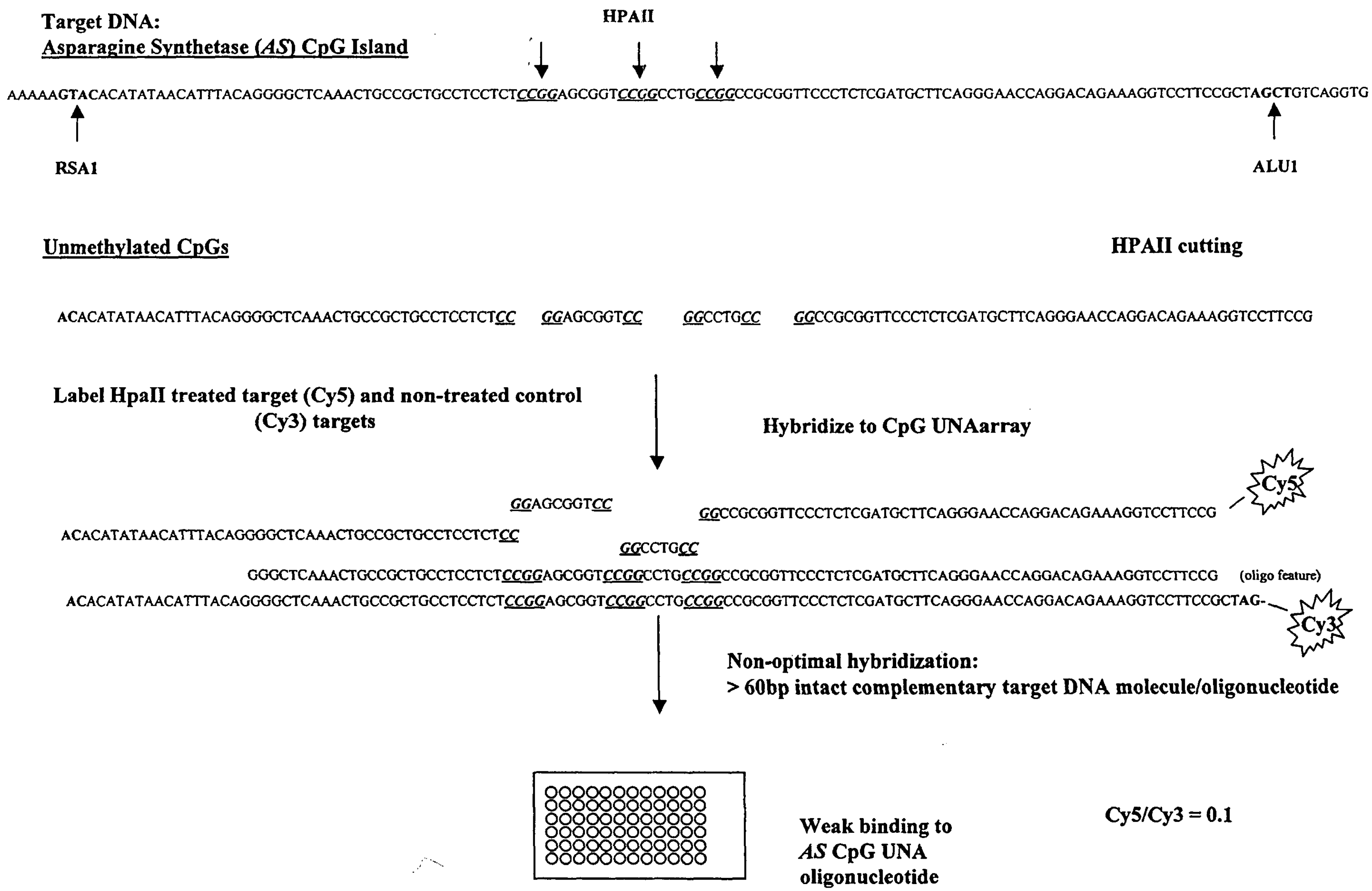
Target DNA:
Asparagine Synthetase (AS) CpG Island
HPAII
AAAAAGTACACATATAACATTTACAGGGGCTCAAACTGCCGCTGCCTCCTCTCCGGAGCGGTCCGGCCTGCCGGCCGCGGTTCCCTCTCGATGCTTCAGGGAACCAGGACAGAAAGGTCCTTCCGCTAGCTGTCAGGTG
RSA1
ALU1
Unmethylated CpGs
HPAII cutting
ACACATATAACATTTACAGGGGCTCAAACTGCCGCTGCCTCCTCTCC GGAGCGGTCC GGCCTGCC GGCCGCGGTTCCCTCTCGATGCTTCAGGGAACCAGGACAGAAAGGTCCTTCCG
Label HpaII treated target (Cy5) and non-treated control (Cy3) targets
Hybridize to CpG UNAarray
GGAGCGGTCC
GGCCGCGGTTCCCTCTCGATGCTTCAGGGAACCAGGACAGAAAGGTCCTTCCG
Cy5
ACACATATAACATTTACAGGGGCTCAAACTGCCGCTGCCTCCTCTCC
GGCCTGCC
GGGCTCAAACTGCCGCTGCCTCCTCTCCGGAGCGGTCCGGCCTGCCGGCCGCGGTTCCCTCTCGATGCTTCAGGGAACCAGGACAGAAAGGTCCTTCCG (oligo feature)
ACACATATAACATTTACAGGGGCTCAAACTGCCGCTGCCTCCTCTCCGGAGCGGTCCGGCCTGCCGGCCGCGGTTCCCTCTCGATGCTTCAGGGAACCAGGACAGAAAGGTCCTTCCGCTAG-
Cy3
Non-optimal hybridization:
> 60bp intact complementary target DNA molecule/oligonucleotide
Weak binding to
AS CpG UNA
oligonucleotide
Cy5/Cy3 = 0.1

FIG. 4B